# EUROPEAN PATENT SPECIFICATION

(11) **EP 1 061 859 B1**
(45) Date of publication and mention of the grant of the patent: **11.05.2005**
(21) Application number: 99911096.8
(22) Date of filing: 03.03.1999
(51) Int. Cl.: A61B 17/11

(54) **ANASTOMOSIS DEVICE**
VORRICHTUNG ZUR ANASTOMOSE
DISPOSITIF POUR L'ANASTOMOSE

(30) Priority: 09.03.1998 US 37113; 09.03.1998 US 37109; 09.03.1998 US 37216
(43) Date of publication of application: 27.12.2000
(73) Proprietor: ETHICON, INC., Somerville, NJ 08876 (US)
(72) Inventor: DUHAYLONGSOD, Francis, G., Honolulu, HI 96819 (US); FRENCH, Fritz, Menlo Park, CA 94025 (US); CHAPMAN, Troy, Englewood, CO 80112 (US); NARCISO, Hugh, L., Jr., Mountain View, CA 94043 (US)
(74) Representative: Nicholls, Michael John
(86) International application number: PCT/US1999/004714
(87) International publication number: WO 1999/045852

(56) References cited:
- EP-A- 0 688 544
- EP-A- 0 791 332
- WO-A-95/09584
- WO-A-96/07355
- WO-A-96/25897
- WO-A-97/29716
- WO-A-98/19630
- DE-A- 2 450 877
- US-A- 3 657 744
- US-A- 4 596 728
- US-A- 4 712 551
- US-A- 4 733 665
- US-A- 5 199 951
- US-A- 5 410 016
- US-A- 5 443 497
- US-A- 5 527 355
- US-A- 5 662 712

## Description

### FIELD OF THE INVENTION

The present invention relates generally to devices for performing a vascular anastomosis and, more particularly, to a device for coupling the end of a vessel, such as a coronary bypass graft, to the side wall of a vessel, such as a coronary artery.

### BACKGROUND OF THE INVENTION

A manifestation of coronary artery disease is the build-up of plaque within the inner walls of the coronary arteries, which causes narrowing or complete closure of these arteries, resulting in insufficient blood flow. This deprives the heart muscle of oxygen and nutrients, leading to ischemia, possible myocardial infarction and even death. When angioplasty is excluded from potential treatments, surgery to alleviate this problem is employed and often involves creating an anastomosis between a coronary artery and a graft vessel to restore a blood flow path to essential tissues. An anastomosis is a surgical procedure by which two vascular structures, such as a graft vessel and a coronary artery, are interconnected.

Current methods available for creating an anastomosis include hand suturing the vessels together. Connection of interrupted vessels with stitches has inherent drawbacks. For example, it is difficult to perform and requires great skill and experience on the part of the surgeon due in large part to the extremely small scale of the vessels. For example, the coronary arteries typically have a diameter in the range of between about 1 to 5 mm, and the graft vessels have a diameter on the order of about 1 to 4 mm for an arterial graft such as a thoracic artery, or about 4 to 8 mm for a vein graft such as a saphenous vein. Other drawbacks of connection with stitches are the long duration of the operation, during which period in conventional open-heart coronary artery bypass graft (CABG) surgery the heart is arrested and the patient is maintained under cardioplegic arrest and cardiopulmonary bypass. Cardiopulmonary bypass has been shown to be the cause of many of the complications that have been reported in conventional CABG, such as stroke. The period of cardiopulmonary bypass should be minimized, if not avoided altogether, to reduce patient morbidity.

One approach to coronary artery bypass grafting that avoids cardiopulmonary bypass is performing the suturing procedure on a beating heart in a minimally invasive direct coronary artery bypass graft ("MIDCAB") procedure. At present, however, safe, reproducible, and precise anastomosis between a stenotic coronary artery and a bypass graft vessel presents numerous obstacles including continuous cardiac translational motion which makes meticulous microsurgical placement of graft sutures extremely difficult. The constant translational motion of the heart and bleeding from the opening in the coronary artery hinder precise suture placement in the often tiny coronary vessel.

The above mentioned drawbacks of hand suturing have led to the development of various approaches to stitchless vascular connection or anastomosis which has the advantage of quick and simple execution and undamaged vascular endothelium. Some approaches to stitchless anastomosis use rigid rings prepared from various materials. For example, Geotz et al., INTERNAL MAMMARY-CORONARY ARTERY ANASTOMOSIS - A Nonsuture Method Employing Tantalum Rings, J. Thoracic and Cardiovasc. Surg. Vol. 41 No. 3, 1961, pp. 378-386, discloses a method for joining blood vessels together using polished siliconized tantalum rings which are circumferentially grooved. The free end of the internal thoracic artery is passed through a ring chosen according to the size of the stenotic coronary artery. The free end of the thoracic artery is everted over one end of the ring as a cuff and fixed with a silk ligature which is tied around the most proximal of the circular grooves in the ring. The cuffed internal thoracic artery is inserted into an incision in the target coronary artery. The ring is fixed in place and sealingly joined to the target coronary artery by tying one or more sutures circumferentially around the target vessel and into one or more circular grooves in the ring. An intima-to-intima anastomosis results.

The use of metallic coupling rings is also disclosed in Carter et al., Direct Nonsuture Coronary Artery Anastomosis in the Dog, Annals of Surgery, Volume 148, No. 2, 1958, pp. 212-218 (describing use of rigid polyethylene rings for stitchless vascular connections). Moreover, for example, U.S. Patent No. 4,624,257 to Berggren et al. describes a device consisting of a pair of rigid rings each having a central opening through which the end of the coronary or graft vessel is drawn and everted over the rings. A set of sharp pins extends outwardly from the face of each ring and pierce through the vessel wall in the everted configuration. The rings are then joined together to align the end of the graft vessel with the opening in the target vessel.

However, no permanently satisfactory results have been reported with the use of rigid rings. A rigid ring presents a foreign body of relatively heavy weight which does not heal well and produces pressure necrosis. Moreover, the use of rigid rings that completely encircle the graft vessel and the arteriotomy creates a severe "compliance mismatch" relative to both the coronary artery and the graft vessel at the anastomosis site which could lead to thrombosis. That is, recent studies suggest that the anastomosis site should not be dramatically different in compliance relative to either the coronary artery or the vascular graft, which is the case when using rigid rings to sealingly join two vessels together.

Another method currently available for stitchless anastomosis involves the use of stapling devices. These instruments are not easily adaptable for use in vascular anastomosis. It is often difficult to manipulate these devices through the vessels without inadvertently piercing a side wall of the vessel. Moreover, as noted above, the scale of the vessels is extremely small, and it is extremely difficult to construct a stapling device that can work reliably on such a small scale to provide a consistent and precise leak-free vascular anastomosis.

In response to the inherent drawbacks of previous devices and methods for performing vascular anastomoses, the applicants have invented novel devices and methods for anastomosing vessels. The methods are not, however, the subjet-matter of patent claims. One method uses deformable or curable materials, which can be molded in vivo to create a shaped article which is capable of sealingly joining a graft vessel to a target vessel in a patent, compliant anastomosis. The application of deformable materials to body tissues of humans to treat various medical conditions has become increasingly important in medicine. By "deformable," it is meant that the material may be transformed from a solid, non-fluent state to a moldable, fluent state in vivo upon the application of energy, such as light energy or heat, to the material. The deformable material, for example, may become moldable in vivo by a heat-activated process upon the application of radiant energy from an energy source such as a radio frequency energy source, microwave energy source, ultrasonic energy source, or light energy source at a predetermined frequency, wavelength or wavelengths. Alternatively, the deformable material may become moldable by other conventional heat-activated heating means, such as by conductive heating or convective heating. In addition, deformable materials that become moldable by a non-thermal light-activated process without generating heat also are generally known. Such materials can be converted to a moldable, fluent state by any one of a number of light-activated processes, such as a photochemical process or a photophysical process (i.e., photoacoustic or plasma formation).

Alternatively, it is also generally known to use curable materials, such as an acrylate or an acrylated urethane material, to bond two materials together, such as body tissue surfaces. A "curable" material refers to a material that can be transformed from a generally fluent, or liquid state, to a solid, non-fluent, cured state upon the application of energy, such as light energy or heat, to the material. The curable material is preferably applied to an internal tissue surface in fluent form, as a liquid or viscous gel. The coated tissue can then be exposed to light, such as ultraviolet, infrared or visible light, or heat, to cure the material and render it non-fluent, in situ. If light is used as the activating medium, the light is selected to be of an appropriate wavelength and intensify to effectively transform the material from its fluent state into its non-fluent state. Heat curable materials can be used in a similar fashion with the method of heating chosen from the list set forth above for deformable materials.

Among the various uses of deformable and curable materials are the prevention of post-operative adhesions, the protection of internal luminal tissue surfaces, the local application of biologically active species, and the controlled release of biologically active agents to achieve local and systemic effects. They may also be used as temporary or long-term tissue adhesives or as materials for filling voids in biological materials. The materials and conditions of application are selected to enhance desirable properties such as good tissue adherence without adverse tissue reaction, non-toxicity, good biocompatibility, biodegradability, and ease of application. Numerous examples of these materials and their various current uses are fully disclosed in United States Patent Nos. 5,410,016 to Hubbell et al. and 5,662,712 to Pathak et al.. However, it is believed that these materials have not been applied to the field of coronary artery bypass graft surgery, and more particularly, to performing vascular anastomoses.

US-A-5,443,497 discloses a tubular prosthetic device for use in coronary bypass surgery which is in accordance with the pre-characterising portion of claim 1. It comprises a tubular member which may be expandable and collapsible, e.g. by being made of mesh, which has one end inserted into an artery and the other end projects out of an opening in the side of the artery and connects to a PTFE (or similar material) bypass tube.

The present invention involves improvements to devices for performing vascular anastomoses. The invention facilitates positioning one vessel in the fluid path of another vessel to enhance the fluid flow juncture therebetween.

According to the present invention there is provided an anastomosis device for use in coupling an end of a first vessel to a side of a second vessel, the second vessel having an opening formed in a side wall thereof for insertion of the device, the device comprising a tubular member at least a portion thereof being radially expandable, the tubular member being preformed with a bend along its central longitudinal axis so that a portion of the tubular member extends out from the opening in the side wall of the second vessel while an end portion of the tubular member extends generally coaxially with the second vessel when the tubular member is inserted into the second vessel, the tubular member being sufficiently rigid in its preformed configuration to substantially retain its bent shape after the tubular member is expanded.

The invention will be further described by way of example with reference to the accompanying drawings, in which:-
Fig. 1A is an elevated view of an anastomosis device of the present invention.
Fig. 1B is an elevated view of the anastomosis device of Fig. 1A with a smooth end margin at one end.
Fig. 1C is an elevated view of a modified configuration of the anastomosis device of Fig. 1A.
Fig. 2 shows the anastomosis device of Fig. 1A connected to a free end of a graft vessel prior to insertion into an artery.
Fig. 3A is an elevated view of the anastomosis device of Fig. 1A with the graft vessel inserted therein.
Fig. 3B is an elevated view of the anastomosis device of Fig. 1B with the graft vessel inserted therein.
Fig. 4 is an elevated view of the anastomosis device of Fig. 3A with a free end of the graft vessel everted over an end of the device.
Fig. 5A is an elevated view of the anastomosis device of Fig. 4 shown in a compressed state for insertion into the artery.
Fig. 5B is a cross-sectional view taken in the plane including line 5B--5B of Fig. 5A.
Fig. 6 shows a balloon catheter ready for insertion into the device of Fig. 5.
Fig. 7 shows the anastomosis device and balloon catheter being inserted into the artery.
Fig. 8 shows the device of Fig. 1A in a completed anastomosis.
Fig. 9 is an elevated view of a second embodiment of an anastomosis device constructed according to the principles of the present invention.
Fig. 10 shows the anastomosis device of Fig. 9 attached to a graft vessel and inserted into an artery.
Fig. 11 is an elevated view of a third embodiment of an anastomosis device constructed according to the principles of the present invention.
Fig. 12 shows the anastomosis device of Fig. 11 attached to a graft vessel and inserted into an artery with a balloon catheter extending through the graft vessel and device and expanding the device.
Fig. 13 shows the device of Fig. 11 in a completed anastomosis.
Fig. 14 is an elevated view of a fourth embodiment of an anastomosis device constructed according to the principles of the present invention.
Fig. 15 is an elevated view of the anastomosis device of Fig. 14 with a vessel inserted therethrough.
Fig. 16 is an elevated view of the anastomosis device of Fig. 15 with a graft vessel connected thereto.
Fig. 17 shows an artery having a balloon catheter inserted therein.
Fig. 18 shows the device of Fig. 16 being placed over the balloon catheter.
Fig. 19 shows the device of Fig. 16 and the balloon catheter being inserted into the artery.
Fig. 20 shows the device and balloon catheter of Fig. 19 positioned within the artery.
Fig. 21 shows the completed anastomosis.
Fig. 22 shows an insert interposed between the artery and the device of Fig. 11.
Fig. 23A is an elevated view of a fifth embodiment of an anastomosis device constructed according to the principles of the present invention inserted into a graft vessel.
Fig. 23B is a cross-sectional view taken in the plane including line 23B--23B of Fig. 23A.
Fig. 24 is a perspective view of an alternative embodiment of an anastomosis device of the present invention showing a graft coupling member coupled to an end of a tubular member.
Fig. 25 is a cross-sectional view of the anastomosis device of Fig. 24 taken through the line A--A of Fig. 24.
Fig. 26 shows the anastomosis device of Fig. 24 prior to insertion of the device into an opening in an introducer.
Fig. 27 shows the anastomosis device of Fig. 24 prior to insertion of the device into an opening in an alternative embodiment of the introducer of Fig. 26.
Fig. 28 is an elevated view of the anastomosis device of Fig. 26 with a free end of a graft vessel inserted therein.
Fig. 29 is an elevated view of the anastomosis device of Fig. 28 with the device inserted into the introducer showing the graft coupling member in a compressed state within the introducer.
Fig. 30 is an elevated view of the anastomosis device of Fig. 29 with the free end of the graft vessel shown extending longitudinally from an end of the introducer.
Fig. 31 is an elevated view of the anastomosis device of Fig. 30 with the free end of the graft vessel everted over an end of the introducer.
Fig. 32 is an elevated view of the anastomosis device of Fig. 31 showing a portion of the graft coupling member extending longitudinally from an end of the introducer and showing the application of one or more sutures between the everted graft vessel and the graft coupling member.
Fig. 33 is an elevated view of the anastomosis device of Fig. 24 shown inserted into the introducer of Fig. 27.
Fig. 34 is an elevated view of the anastomosis device of Fig. 32 inserted into a target vessel through an incision in the target vessel.
Fig. 35 is an elevated view of the anastomosis device of Fig. 34 after the introducer has been removed from the fastener showing the completed anastomosis.
Fig. 36 is an alternative embodiment of the anastomosis device of Fig. 24.
Fig. 37 shows the anastomosis device of Fig. 36 with a graft vessel inserted therein prior to insertion of the device into an opening in the tubular introducer.
Fig. 38 is an elevated view of the anastomosis device of Fig. 37 with the device inserted into the introducer and showing the tubular member in a compressed state within the introducer.
Fig. 39 is an elevated view of the anastomosis device of Fig. 38 with the free end of the graft vessel everted over an end of the introducer.
Fig. 40 is an elevated view of the anastomosis device of Fig. 39 showing a portion of the tubular member extending longitudinally from an end of the introducer and showing the application of one or more sutures between the everted graft vessel and the tubular member.
Fig. 41 is an elevated view of the anastomosis device of Fig. 40 inserted into the target vessel through an incision in the target vessel.
Fig. 42 is an elevated view of the anastomosis device of Fig. 41 after the introducer has been removed from the fastener showing the completed anastomosis.
Fig. 43 is a perspective schematic view of an alternative embodiment of an anastomosis device of the present invention showing a formable, moldable tubular member.
Fig. 44 is a perspective schematic view of an alternative embodiment of the anastomosis device of Fig. 43 showing a thin sheet of formable, moldable material.
Fig. 45 shows a pre-formed tubular member.
Fig. 46 shows the anastomosis device of Fig. 43 after positioning the device about an external surface of a free end of the graft vessel.
Fig. 47 is an elevated view of the anastomosis device of Fig. 46 with the free end of the graft vessel everted over a portion of the tubular member.
Fig. 48 is an elevated view of the anastomosis device and graft vessel of Fig. 47 and a light-diffusing balloon catheter prior to insertion of the catheter longitudinally into the graft vessel.
Fig. 49 is an elevated view of the anastomosis device of Fig. 48 and balloon catheter inserted into the target vessel through an incision in the target vessel.
Fig. 50 is an elevated view of the anastomosis device of Fig. 49 following light irradiation and radial expansion of the balloon.
Fig. 51 is an elevated view of the anastomosis device of Fig. 50 after the light-diffusing balloon catheter has been removed from the graft vessel showing the completed anastomosis.
Fig. 52 is an elevated view of an alternative embodiment of an anastomosis device prior to insertion into a graft vessel.
Fig. 53 shows the anastomosis device of Fig. 52 after insertion of the device into the graft vessel.
Fig. 54 is an elevated view of the anastomosis device of Fig. 52 with a light-diffusing balloon catheter inserted into the graft vessel and the device.
Fig. 55 is an elevated view of the anastomosis device, graft vessel and catheter of Fig. 54 inserted into the target vessel through an incision in the target vessel.
Fig. 56 is an elevated view of the anastomosis device of Fig. 55 following light irradiation and expansion of the balloon.
Fig. 57 is an elevated view of the anastomosis device of Fig. 56 after the light-diffusing balloon catheter has been removed from the graft vessel showing the completed anastomosis.

Corresponding reference characters indicate corresponding parts throughout the several views of the drawings. It should be noted that certain embodiments do not comprise all features as defined in claim 1, but still help to illustrate and understand the invention.

### DESCRIPTION OF THE INVENTION

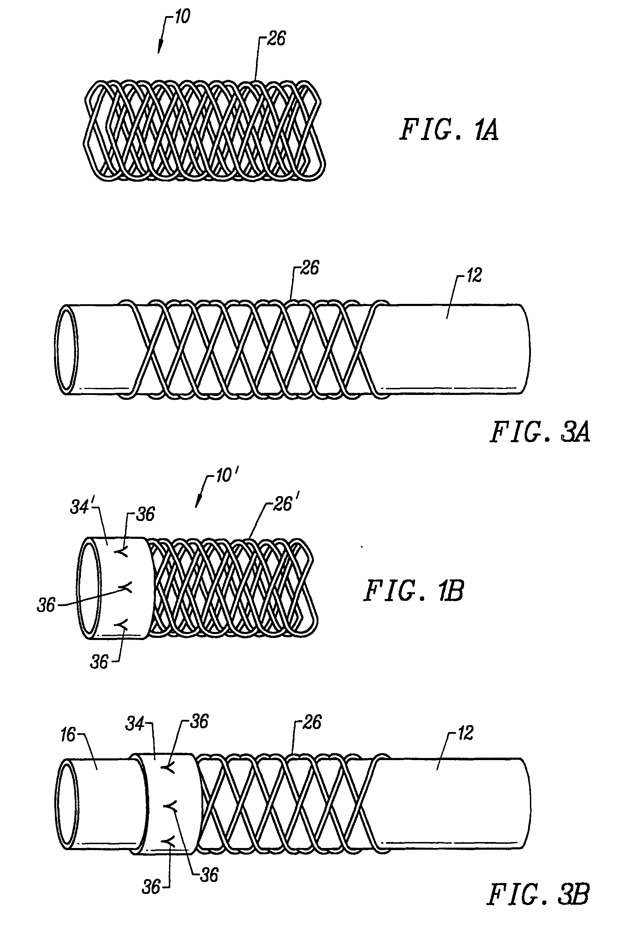

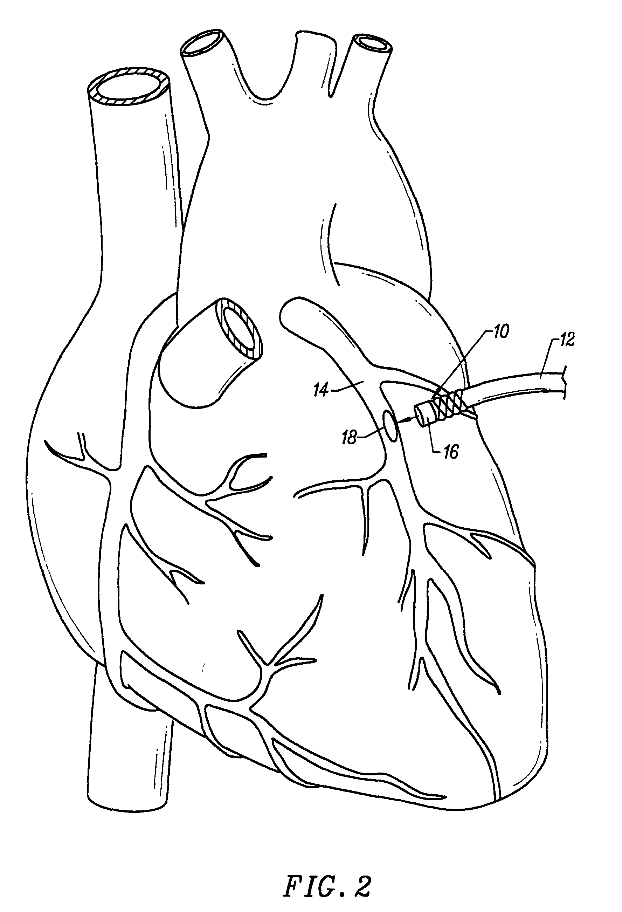

Referring now to the drawings, and first to Fig. 1A, an anastomosis device constructed according to the principles of the present invention is shown and generally indicated with reference numeral 10. The anastomosis device (or fastener) 10 is used to connect a first vessel 12, such as a graft vessel or a thoracic artery to a second vessel 14, such as a coronary artery or vein (Fig. 2). The anastomosis device 10 of the present invention may also be used in connecting various other vessels or arteries and may be used to connect synthetic vascular grafts to an artery.

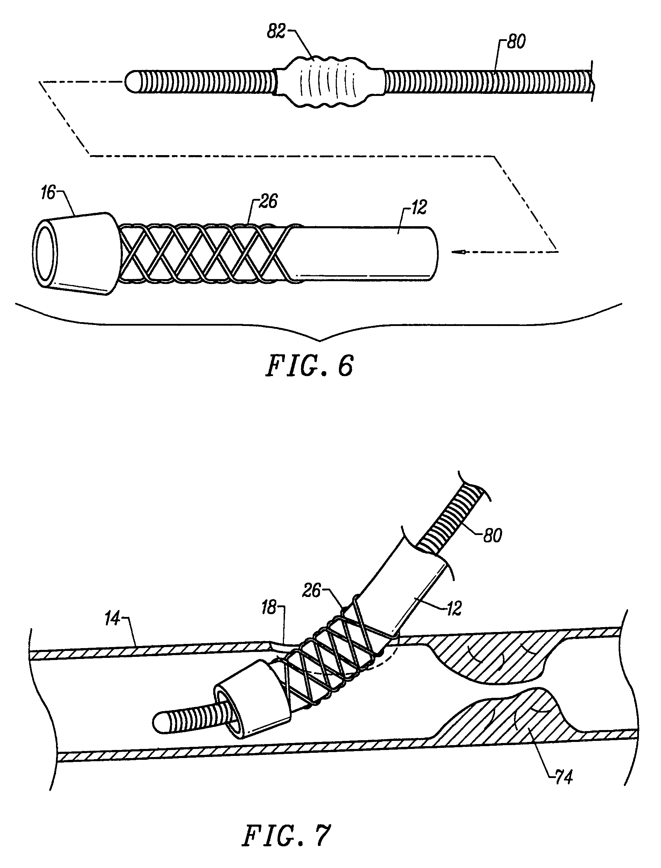

The fastener 10 comprises a tubular member 26. The tubular member 26 may be formed from wire as shown in Fig. 1A. The fastener is preferably formed from a mesh material so that the tubular member 26 is sufficiently radially rigid to maintain the fastener 10 in a compressed state, yet flexible enough to be inserted through an opening 18 in the side wall of the vessel 14 (Fig. 7). A rigid coil or cage type member may also be used. The tubular member 26 is preferably between about 4 and 12 mm in length, and more preferably between about 5 and 8 mm, for example. The diameter of the fastener 10 in its free state (not compressed or expanded) is preferably between. 1 and 6 mm, for example. The tubular member 26 may also have graft material (not shown) attached to it.

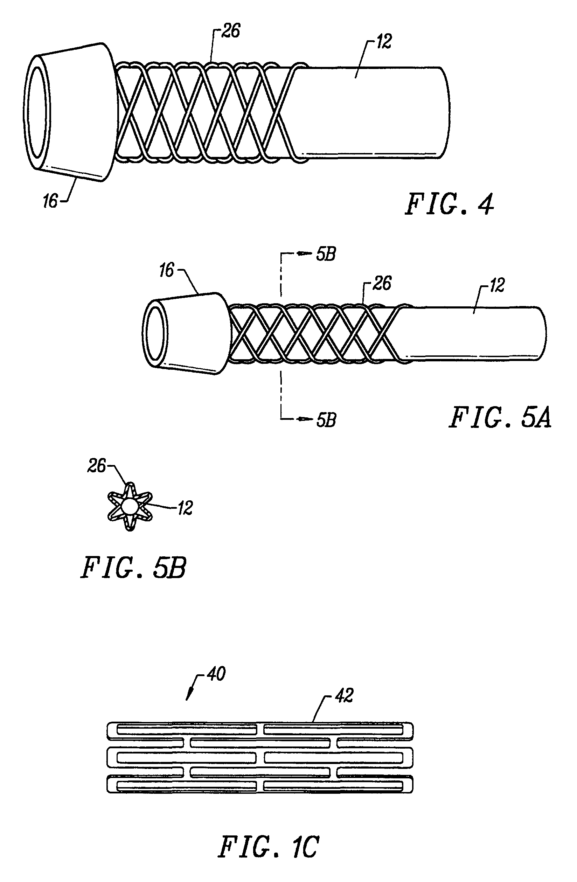

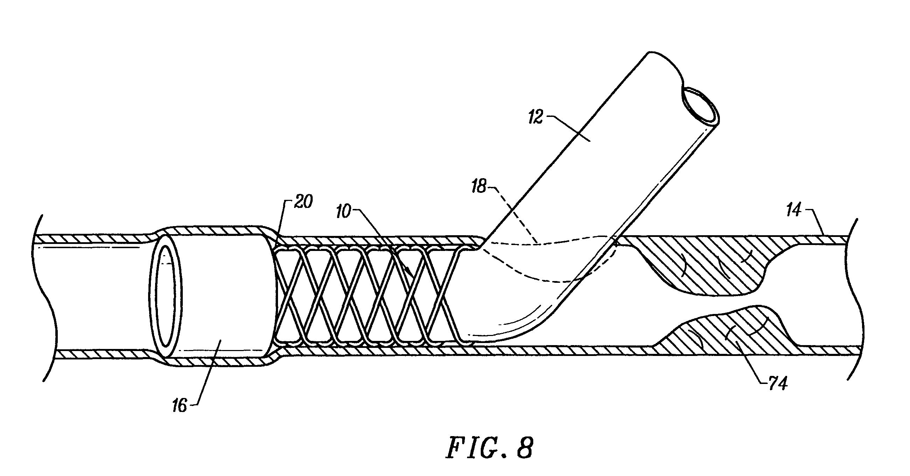

The fastener is radially compressible and expandable so that the fastener is transformable between a compressed state (Figs 5A and 5B) and an expanded state (Fig. 8). When used in a distal anastomosis within the coronary artery, for example, the fastener 10 is preferably constructed with the following characteristics. In its compressed state, the fastener 10 is radially compressed to reduce the outer diameter of the fastener to less than about 2 mm. The diameter of the fastener 10 in its compressed state must be smaller than the opening 18 in the side wall of the artery 14 and smaller than the inner diameter of the artery to permit the fastener to be inserted through the opening and moved longitudinally through the artery to be properly positioned (Fig. 7). The tubular member 26 is sufficiently rigid in the axial direction to remain in its compressed state while being inserted into the artery 14 without a sheath or other device radially restraining the fastener. In its expanded state, the outer diameter of the fastener 10 is at least equal to the inner diameter of the artery 14 so that the graft vessel 12 is in sealing engagement with the inner wall of the artery (Fig. 8). The outer diameter of the fastener 10 in its expanded state is approximately 2-4 mm, and is dependent on the inner diameter of the artery 14.

The tubular member 26 may be formed from stainless steel, tantalum, gold, titanium, shape memory alloys such as nitinol, or any other suitable biocompatible material. The tubular member 26 may also be formed from polymeric materials which satisfy the requisite strength and flexibility requirements described above. It is to be understood that other types of tubular members and different sizes of members or materials may be used without departing from the scope of the invention.

A modified configuration of the anastomosis device 10 of Fig. 1A is shown in Fig. 1B and generally indicated at 10'. The tubular member 26' has an end margin 34' extending from one end thereof. The end margin 34' has a substantially smooth outer surface and may be formed from a continuous piece of metal or any other suitable expandable material. The material is preferably substantially impermeable to blood, to prevent blood from flowing transversely through the end margin 34'. A plurality of barbs 36 extends radially outward from the end margin 34' to securely attach the graft vessel 12 to the fastener 10' (Fig. 3B). The barbs 36 pierce the wall of an everted end 16 of the graft vessel 12 to securely hold the graft vessel in place on the fastener 10. The tubular member 26 may also be formed without the smooth end margin 34', with the barbs 36 connected directly to the member. The barbs 36 may be attached to the tubular member 26' with sutures, thread, or glue, or welded directly to the member, for example. The barbs 36 may also be eliminated and the everted end 16 of the graft vessel 12 may be held in place with biological glue or other suitable adhesive means.

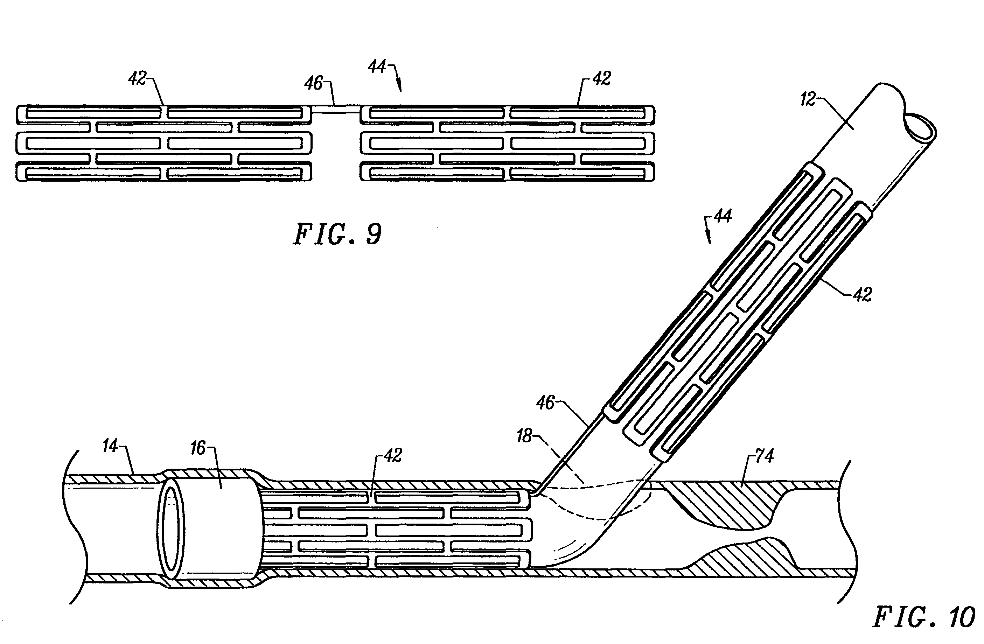

Another modified configuration of the fastener 10 is shown in Fig. 1C and generally indicated at 40. The fastener 40 comprises a tubular member 42. The tubular member 42 may be made from a stent, generally indicated at 44 (Fig. 9), and further described below. The stent 44 comprises two sections hingedly connected together with a hinge 46. In order to form the fastener 40, the stent 44 is cut in half at the location of the hinge with a suitable cutting instrument. The tubular member 42 preferably has the dimensions and characteristics described above for tubular member 26.

A second embodiment of the present invention is the stent 44 shown in Fig. 9. Instead of cutting the stent 44 into two separate sections, the entire stent (tubular member) is attached to the graft vessel 12 and inserted into the artery 14 as shown in Fig. 10. The length of the stent is preferably between 10 and 20 mm, for example. The stent 44 may be a stent available from Johnson & Johnson Interventional Systems, Inc. Warren, New Jersey, under model number series PS153 (commonly known as a Palmaz-Schatz stent), for example. An example of the stent 44 is disclosed in U.S. Patent No. 4,733,665, which is incorporated herein by reference. The hinge 46 is preferably formed from a flexible strip of metal or any other suitable material. The hinge 46 may also be formed from two or more components (not shown) which cooperate to form a hinge.

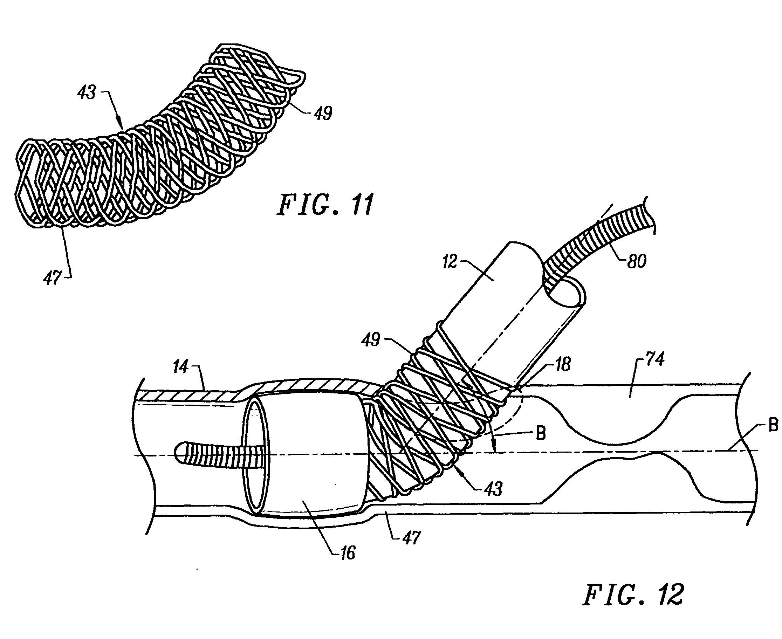

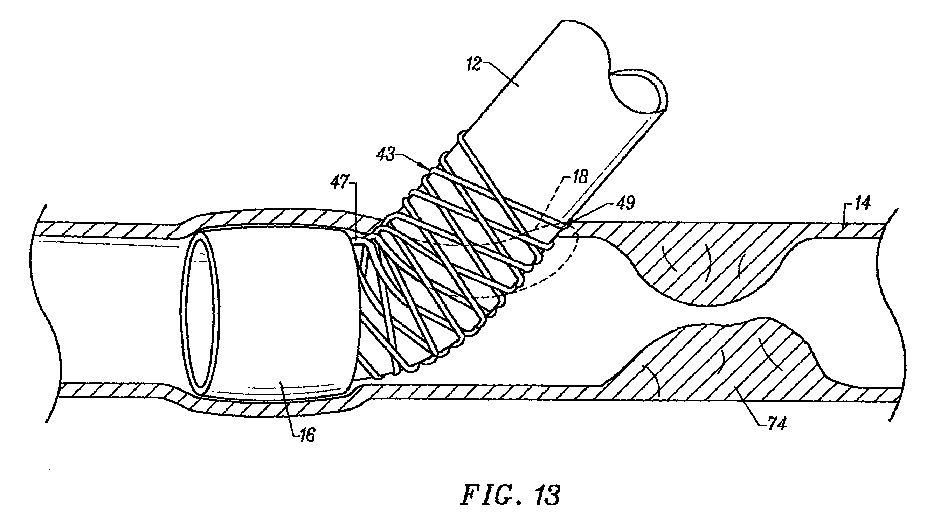

A third embodiment of the present invention is shown in Fig. 11 and generally indicated with reference numeral 43. The fastener 43 comprises a tubular member which is preformed in a bended configuration. The tubular member has a first portion 47 which extends generally straight along a central longitudinal axis B and an angled portion 49 which extends at an angle β of between about 30 and 60 degrees relative to the central longitudinal axis of the first portion. The bent configuration allows the fastener 43 to extend out from the opening 18 in the artery 14 (Figs. 12 and 13). The fastener 43 thus supports the graft vessel 12 through the arteriotomy to prevent kinking of the graft vessel 12 when the fastener 43 is in its expanded state. A side wall of the tubular member preferably extends substantially around the circumference of the tubular member along its entire length to further prevent kinking. The fastener 43 is preferably formed from stainless steel, tantalum, gold, titanium, shape memory alloys such as nitinol, or any other suitable material which can be formed in a bent configuration and retain its shape. The fastener 43 may also be formed from suitable polymeric materials which can be molded into a bent configuration. The length of the fastener 43 is preferably between 6 and 20 mm, for example.

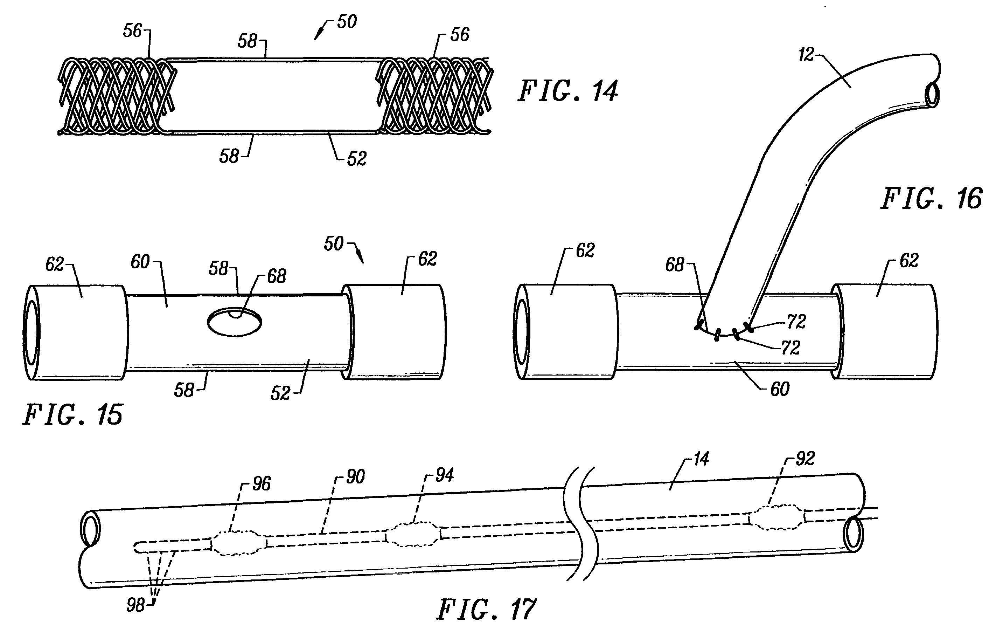

A fourth embodiment of the present invention is shown in Fig. 14 and generally indicated with reference numeral 50. The fastener 50 comprises a tubular member 51 having a central portion 52 and two end portions 56. The central portion 52 comprises a plurality of struts 58 extending longitudinally between the end portions 56. The struts 58 are preferably formed from a stiff material to prevent the tubular member from buckling in the axial direction. The central portion 52 preferably comprises at least two struts 58 to provide sufficient longitudinal stiffness to the fastener 50. The end portions 56 each comprise an expandable ring formed from a mesh or other suitable materials.

As shown in Fig. 15, an elongated (cylindrical) member 60, formed from a vessel having an outer diameter slightly smaller than the inner diameter of the fastener 50, is inserted through a central longitudinal opening 64 of the fastener. The elongated member 60 is preferably formed from an autograft vessel, taken from the patient's body, but may also be formed from a synthetic vessel made of a suitable biologically inert material. The material of the elongated member 60 is preferably substantially nonporous, with respect to blood, to prevent leakage of the anastomosis. The elongated member 60 is preferably longer than the fastener 50 so that both ends 62 of the elongated member can be everted over the ends of the fastener. The end portions 56 of fastener 50 may have barbs (not shown) extending radially outward therefrom for securing the everted ends 62 of the member to the fastener, as described above for the first embodiment 10. With the elongated member 60 in place, the interior surfaces of the fastener 50 are covered with vascular tissue so that a smooth, continuous, hemocompatible layer is exposed to the bloodstream. This reduces the likelihood of hemolysis or thrombosis due to the presence of foreign material in the bloodstream. The length of the fastener 50 may be 10-17 mm, for example, and is preferably between 13 and 15 mm. The end portions 56 of the fastener 50 each have a length of at least 2 mm, for example, to provide sufficient circumferential surface area for engagement with the inner wall of the artery 14, as described further below.

An opening 68 is formed in a side wall of the elongated member 60 at a location along the central portion 52 of the fastener 50. A free end of the graft vessel 12 is attached to the periphery of the opening 68 by sutures 72, glue, mechanical clips or other suitable means. Alternatively, the free end of the graft vessel may have a large transverse artery or vein branch at its distal end (i.e., the graft vessel has a general T-shaped configuration with a marginal branch vessel at its distal end), in which case the branch can be inserted distally into the opening 68 in the target vessel without the need for additional attachment means. The diameter of the opening 68 in the elongated member 60 is preferably sized to correspond to the diameter of the graft vessel 12 and may be 4-5 mm, for example. A suture pad (not shown) formed in the shape of a ring may be attached to the periphery of the opening 68 in the elongated member 60 to prevent tearing of the member. The pad provides reinforcement to the elongated member 60 and prevents the initiation of tears at the opening 68. The fastener 50 is configured to allow bi-directional flow therethrough. The blood enters the elongated member 60 at the opening 68 therein, and flows out from both ends 56 of the fastener in a direction generally transverse to the direction of the flow entering the elongated member.

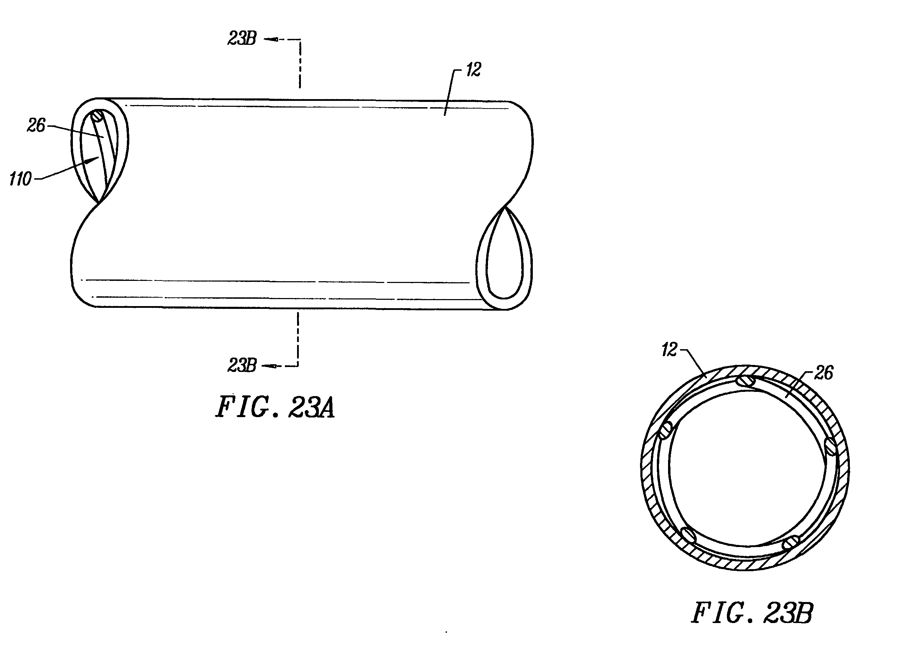

A fifth embodiment of the anastomosis device of the present invention is shown in Figs. 23A and 23B and generally indicated at 110. The fastener 110 is similar to the first embodiment 10, except that the tubular member 26 is disposed within the graft vessel 12 rather than over the vessel. The member 26 is compressed to have an outer diameter smaller than the inner diameter of the vessel 12 and then inserted longitudinally into the vessel 12. The member 26 may be attached to the vessel 12 by sutures or other suitable attachment means (not shown). Instead of using attachment means, the tubular member 26 may be slightly expanded to engage the inner wall of the graft vessel 12 to hold the member in place within the vessel. The tubular member 26 may be expanded with a balloon catheter, for example. The fastener 110 can be completely enclosed by the graft vessel 12 as shown, or can extend a short distance from the end of the graft vessel.

Figure 2 shows an exemplary use of the anastomosis device 10 of the present invention in an open surgical coronary artery bypass graft procedure to create a distal anastomosis. The left internal thoracic artery is used as the graft vessel 12. In this example, the left anterior descending artery 14 contains a blockage or narrowing 74. If left untreated, this diseased artery may lead to insufficient blood flow and eventual angina, ischemia, and possibly myocardial infarction.

Conventional coronary bypass graft procedures require that a source of arterial blood be prepared for subsequent bypass connection to the diseased artery. An arterial graft may be used to provide a source of blood flow, or a free graft may be used and connected at the proximal end to a source of blood flow. Preferably, the source of blood flow is one of any number of existing arteries that are dissected in preparation for the bypass graft procedure. In many instances it is preferred to use either the left or right internal thoracic artery. Other vessels which may be used include the saphenous vein, gastroepiploic artery in the abdomen, radial artery, and other arteries harvested from the patient's body as well as synthetic graft materials, such as Dacron or Goretex grafts. If a free graft vessel is used, the upstream end of the dissected vessel, which is the arterial blood source, will be secured to the aorta to provide the desired bypass blood flow, as is well known by those skilled in the art. It is to be understood that the anastomosis device of the present invention may be used in other vessel anastomoses.

In order to perform an anastomosis with the fasteners 10, 10', 40 of the first embodiment, or the fasteners 44, 43, of the second and third embodiments, respectively, the graft vessel 12 is first inserted into the device with an insertion tool (not shown) as is well known by those skilled in the art (Fig. 3A). The following example refers generally to fastener 10, but applies to the other fasteners of the first embodiment except where noted. The graft vessel 12 is pulled through the fastener 10 until the graft vessel extends a short distance from one end of the device. The free end 16 of the graft vessel 12 is then everted over the end of the fastener 10 (Figs. 3A and 4). The free end may also be placed over the barbs 36 extending from the end margin 34' of the fastener 10' (Fig. 3B). The barbs 36 partially penetrate the wall of the graft vessel 12 to securely hold the vessel in place on the fastener 10'.

After attaching the graft vessel 12 to the fastener 10, the fastener is radially compressed to reduce the outer diameter of the fastener (Figs. 5A and 5B). The diameter is reduced sufficiently to allow the fastener 10 to move longitudinally within the artery 14. A balloon catheter 80 having at least one balloon 82, is inserted through the lumen of the vessel 12 and fastener 10 to expand the end portion 20 of the fastener for engagement with an inner wall of the artery 14 (or vein) (Fig. 6). A slit, approximately 5-10 mm in length is formed in a side wall of the artery 14 with a scalpel or other appropriate cutting instrument. Alternatively, a circular or oval punch may be used to facilitate the arteriotomy. The fastener 10 is then inserted into the opening 18 formed in the vessel (Fig. 7). The fastener 10 is positioned within the artery 14 so that the end portion 20 of the fastener extends generally coaxial with the artery. The fastener 43 is preferably positioned in the artery 14 such that the majority of the fastener is disposed within the artery (e.g., approximately 80 percent of the fastener is located within the artery) (Fig. 13). The balloon 82 is inflated to radially expand the end portion 20 of the fastener 10 so that the graft vessel 12 sealingly engages an inner wall of the artery 14 to secure the fastener within the artery, and prevent leakage of blood between the everted end 16 of the vessel 12 and the inner wall of the artery (Fig. 8). The engagement of the graft vessel 12 with the inner wall of the artery 14 prevents substantial longitudinal movement of the fastener within the artery. The balloon 82 is deflated and the catheter 80 is withdrawn from the graft vessel. The vessel 12 is now coupled with the artery 14 and the anastomosis is complete. If necessary, a biological glue may be coated to the everted surface of the graft vessel to facilitate a fluid-tight seal.

The following example is provided for purposes of illustration and is not intended to limit the invention. Anastomoses were created on cadaver hearts with a fastener constructed as shown in Fig. 1C. The fastener was formed by cutting a stent as shown in Fig. 9 in half. The left internal thoracic artery or saphenous vein were harvested and passed through the length of the fasteners so that a 2 mm to 3 mm cuff extended beyond the end of the fasteners. The cuff was everted around the end of the fastener (Fig. 4). The fastener was then compressed around an angioplasty balloon catheter. A 7-10 mm arteriotomy was performed and the fastener was inserted through the arteriotomy into the coronary artery about 75% to 100% the length of the fastener. The balloon was inflated to 14 atmospheres for 30 seconds. The balloon was then deflated and the catheter was removed, leaving the fastener within the coronary artery. Colored saline was injected into the internal thoracic artery and saphenous vein grafts under high pressure (in excess of 300 mm Hg). In two of nine instances leaks were observed. In one case, the fastener was not seated deeply enough within the coronary vessel (>25% of the length of the fastener). In one additional cadaver, a biologic glue was applied around the everted surface of the graft. In the four instances in which it was tried, no leaks were observed around the fastener.

In order to insert the fastener 50 of the fourth embodiment, a vessel is provided for use as the elongated member 60 (Fig. 15). The elongated member 60 is inserted through the longitudinal opening 64 in the tubular member and the ends 62 of the vessel 60 are everted over the tubular member. An opening 68, having a diameter of approximately 4-5 mm is formed in the side wall of the elongated member 60. A reinforcement ring or suture pad (not shown) may be attached to the periphery of the opening 68 in the side wall of the elongated member 60. The graft vessel 12 is then attached to the tubular member at the location of the opening 68 in the side wall of the elongated member 60 (Fig. 16). A balloon catheter 90, preferably having three balloons 92, 94, 96, is inserted into the artery 14 (Fig. 17). The upstream balloon 92 (balloon farthest to the right as viewed in Fig. 17) is inflated to function as an occlusion catheter and block the flow of blood at the location of the anastomosis. Blood is supplied downstream of the anastomosis through openings 98 located on the end of the catheter 90. The two downstream balloons 94, 96 may be combined into a single cylindrical balloon (not shown) having a length approximately equal to the length of the fastener 50.

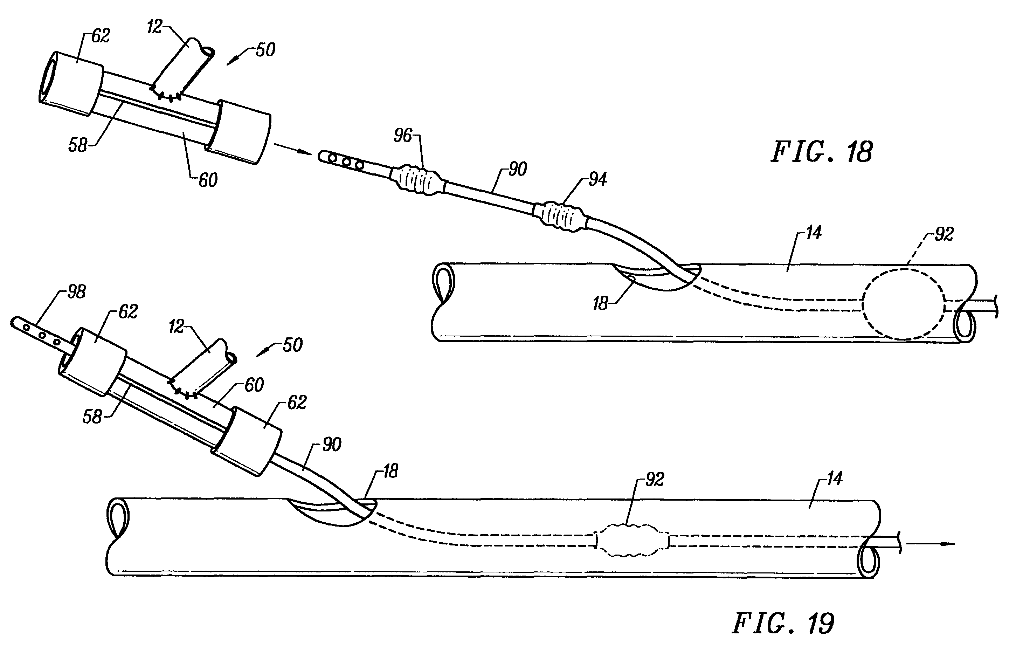

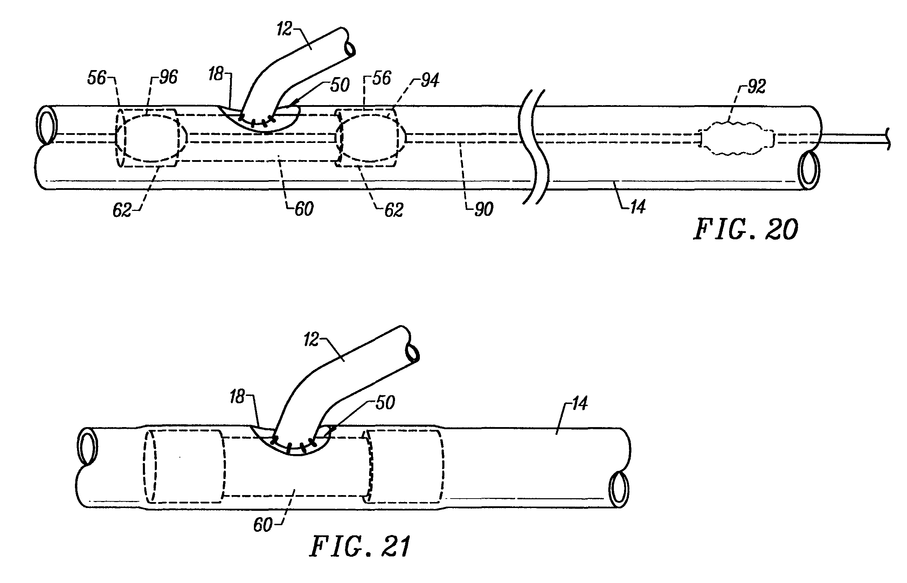

A slit, approximately 10-15 mm in length is formed in the side wall of the artery 14 at a location between two of the balloons located at the end of the catheter to form the opening 18 (Fig. 18). The downstream end of the catheter 90 is pulled through the opening 18 in the side wall of the artery 14 and inserted through the fastener 50 (Fig. 19). The fastener 50 and catheter 90 are then inserted through the opening 18 into the artery 14 with the fastener extending generally longitudinally along the artery and the graft vessel 12 extending through the opening generally transversely to the fastener (Fig. 20). In the alternative, the catheter 90 can remain in the artery 14 while the fastener 50 is inserted into the artery and placed over the catheter. The fastener 50 is positioned so that the graft vessel 12 extends through generally the center of the opening 18 and the end portions 56 of the fastener are located on opposite sides of the opening. The two downstream balloons 94, 96 are inflated and the end portions 56 are expanded to engage the inner walls of the artery 14. The balloons 92, 94, 96 are then deflated and the catheter 90 is removed from the artery 14 to form the completed anastomosis (Fig. 21).

The fastener 110 of the fifth embodiment (Fig. 23A) is inserted into the second vessel as described above for the first embodiment, after insertion of the fastener into the graft vessel 12.

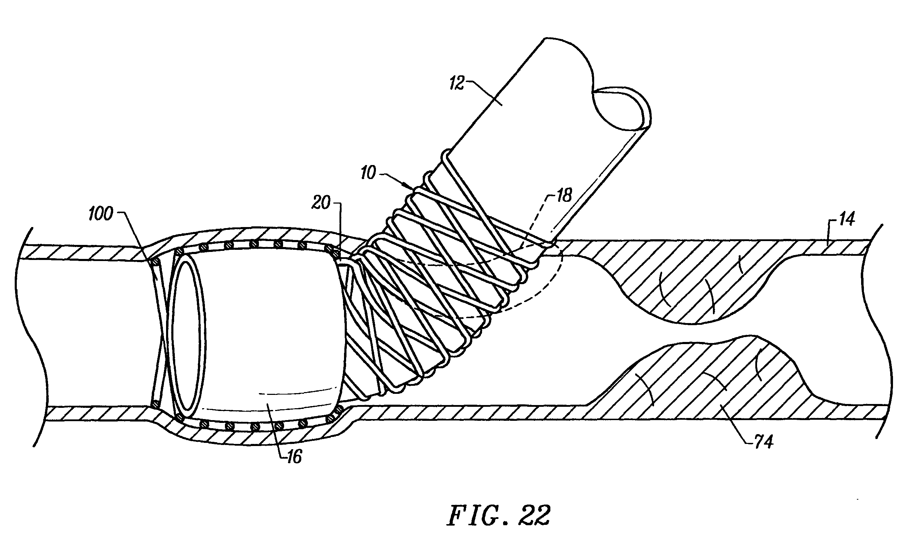

A second insert 100 may also be inserted into the artery and expanded to dilate the artery 14 prior to inserting the fastener of the first 10, 10', 40, second 44, third 43, fourth 50, or fifth 110 embodiments. Use of an insert with fastener 43 is shown in Fig. 22. The insert 100 is inserted into the artery 14 at a location where the portion of the fastener 43 to be expanded will be located. The fastener 43 is then inserted into the artery 14 and insert 100, and expanded. Two inserts 100 may similarly be inserted into the artery 14 at the locations where each end of the fastener will be placed for fastener 50. The insert 100 may be formed of a mesh material as described above.

The anastomosis devices may also be expanded without the use of balloons. For example, the tubular member may be formed of a shape memory alloy such as nitinol, as is well known by those skilled in the art. After the fastener is attached to the graft vessel 12, the tubular member is cooled and reshaped to a compressed form. The fastener is then inserted into an insulated sheath (not shown) to maintain the temperature of the tubular member below its transformation temperature. The sheath is then inserted and properly positioned within the artery 14. The sheath is removed and the tubular member is permitted to be warmed above its transformation point and urged against the inner wall of the artery 14.

If required, cardiac stabilization such as described in co-pending patent application, serial number 09/131,075 for Compositions, Apparatus and Methods For Facilitating Surgical Procedures, filed August 7, 1998 and invented by Francis G. Duhaylongsod, MD., may be used during the procedure. Other pharmacological or mechanical methods may also be used.

The anastomosis devices of the first, second, third and fifth embodiments may be supplied alone or with a prosthetic graft vessel already attached to the fastener. The anastomosis device 50 of the fourth embodiment may be supplied as a tubular member by itself, a tubular member with the elongated member 60 already inserted, or a tubular member with the elongated member inserted and a prosthetic graft vessel attached to the elongated member.

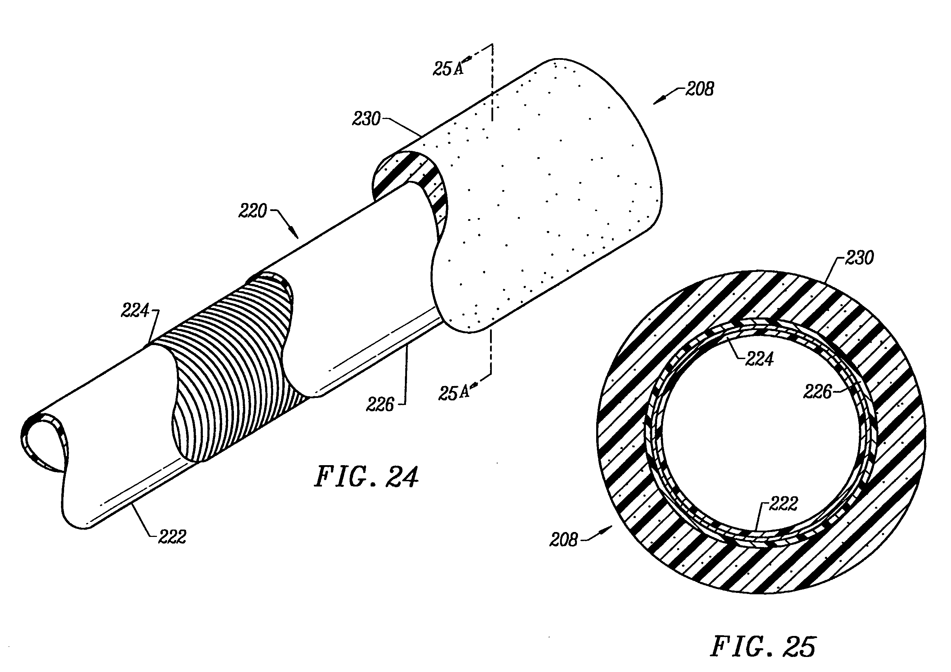

Referring now to Figs. 24-35, an alternate embodiment of the anastomosis device is shown and generally indicated with reference numeral 208. The anastomosis device (or fastener) 208 is used to connect a graft vessel 210, such as a thoracic artery, to a target vessel 212, such as a coronary artery, i.e., the left anterior descending artery. For example, the device may be used in an end-to-side distal anastomosis to sealingly join a thoracic artery or a saphenous vein graft to a coronary artery. The anastomosis device 208 of the present invention may also be used in connecting various other vessels or arteries and may be used to connect synthetic vascular grafts to an artery.

The fastener 208 preferably comprises an elongated, flexible tubular member 220 and a radially compressible, elongated graft coupling member 230 which is coupled to an external surface of the tubular member 220 at one end of the tubular member 220. The graft coupling member 230 can be coupled to the tubular member 220 with biological glue, other adhesive means, thread, or by any other suitable means. Alternatively, the graft coupling member 230 and the tubular member 220 can also be provided as separate parts. The graft coupling member 230 will be configured to be stretched over one end of the tubular member 220 and secured proximal to the end of the tubular member 220 by the force of its own natural compressibility or by any of the coupling means described above.

The tubular member 220 comprises two tubular layers 222, 226, and a thin flexible central tube 224 interposed between the two tubular layers. The tubular layers are preferably formed from a biocompatible, implantable plastic material. The central tube 224 is preferably formed from a biocompatible coil such as medical grade stainless steel or nitinol wire or ribbon, but may also be formed from any other sufficiently rigid, biocompatible material such as a plastic, polyurethane or polycarbonate material, or any other suitable material as is well known to a person of ordinary skill in the art. The inner and outer tubular layers 222, 226 are preferably formed from a material having a low durometer hardness to provide sufficient flexibility to allow the tubular member to bend at an angle of approximately 30° to 60° along a central longitudinal axis of the member. Preferably, the inner and outer layers 222, 226 are made from an implantable, flexible plastic such as silicone, although any other suitable flexible, implantable biocompatible material as is well known to persons of ordinary skill in the art may be used. The inner and outer layers 222, 226 are preferably bonded together (similar to braided shafts) by conventional means and the central tube 224 is locked between the two layers.

The inner plastic tube 222 is generally necessary to prevent the central coil 224 from scraping and damaging the graft vessel 210. The central coil 224 provides strength to the tubular member 220 to prevent it from kinking, yet is sufficiently flexible to allow the tubular member 220 to be inserted into the target vessel 212 through an incision 216 formed therein. The outer plastic tubular layer 226 allows the graft coupling member 230 to be more easily fitted over the tubular member 220 and coupled thereto. The tubular member 220 is preferably between about 4.0 and 12.0 mm in length, and more preferably about 5.0 to 8.0 mm, for example. As shown in Fig. 24, the inner and outer layers 222, 226 and central coil 224 preferably extend the full length of the tubular member 220. The diameter of the tubular member 220 will vary depending on the size of the graft vessel into which it is inserted. Preferably, the inner diameter of the inner layer 222 will generally be between about 0.5 to 6.0 mm for a coronary anastomosis, for example. The total wall thickness of tubular member 220 will be between about 0.100 mm and 0.600 mm, and preferably between about 0.100 mm and 0.400 mm, for example. It is to be understood, however, that other types of tubular members made from other types of biocompatible materials and different size tubular members may be used without departing from the scope of the invention. For example, the tubular member 220 may be formed from a one-piece flexible tube (not shown).

The graft coupling member (compressible portion) 230 comprises a tube formed from a biocompatible, radially compressible material. By "radially compressible", it is meant that the graft coupling member 230 is generally uniformly radially transformable between a free, normal expanded state and one or more compressed states in which the graft coupling member 230 has a smaller diameter than in its normal expanded state. In a preferred embodiment, the biocompatible material comprises a non-metallic foam material which is radially self-expandable. By "self-expandable", it is meant that the foam material forming the graft tubular member 230 is biased to its expanded state (i.e., will naturally tend to radially migrate back towards its free, normal expanded state from its compressed state). The coupling member 230 has an inner surface and an outer surface defining a wall thickness therebetween. The wall thickness of the coupling member in its compressed state is less than the wall thickness of the member in its expanded state. The foam material may be conventional biocompatible foam such as 100 pores per inch foam material, for example. The foam material is substantially radially compressible to allow the graft vessel 210 to be coupled to the foam and to permit the fastener 208 to move longitudinally within the target vessel 212 as will be described in greater detail below. The diameter of the graft coupling member 230 will vary depending on the size of the target vessel 212 into which the fastener 208 is inserted. Preferably, the inner diameter of the graft coupling member 230 will be about 10 to 30% smaller than the outside diameter of the tubular member 220, and the outside diameter of graft coupling member 230 will preferably be between about 10 to 80% larger than an inside diameter of the target vessel 212.

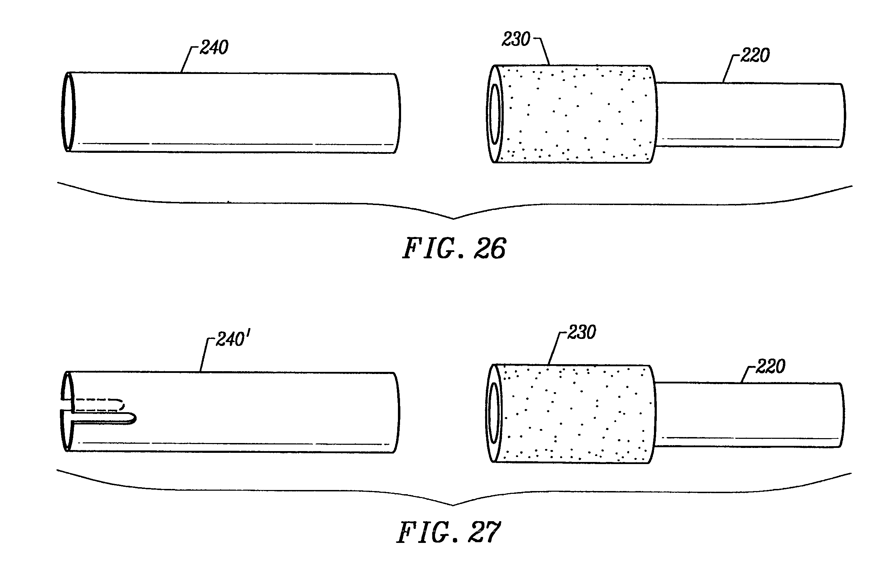

Figures 26-35 show an exemplary use of the anastomosis device 208 of the present invention in an open surgical coronary artery bypass graft procedure via a median sternotomy. This example is meant to be by illustration only, and in no way is meant to be limiting. The present invention can be used in other cardiac surgery procedures such as minimally invasive direct coronary artery bypass grafting (MIDCAB) on a beating heart through a small incision (thoracotomy) (about 6-8 cm) in the left side of the chest wall, in endoscopic minimally invasive cardiac surgery bypass graft procedures, and in other vascular procedures to join two vessels together. By way of example, the left internal thoracic artery is used as the graft vessel 210. In this example, the left anterior descending artery is used as the target vessel 212 and contains a build-up of plaque or narrowing 213. If left untreated, this diseased artery may lead to insufficient blood flow and eventual angina, ischemia, and possibly myocardial infarction.

Conventional coronary bypass graft procedures require that a source of arterial blood be prepared for subsequent bypass connection to the diseased artery. An arterial graft can be used to provide a source of blood flow, or a free vessel graft may be used and connected at the proximal end to a source of blood flow. Preferably, the source of blood flow is any one of a number of existing arteries that are dissected in preparation for the bypass graft procedure. In many instances, it is preferred to use either the left or right internal thoracic artery. In multiple bypass procedures, it may be necessary to use free graft vessels such as the saphenous vein, gastroepiploic artery in the abdomen, and other arteries harvested from the patient's body as well as synthetic graft materials, such as Dacron or Gortex grafts. If a free graft vessel is used, the upstream end (proximal) of the dissected vessel, which is the arterial blood source, will be secured to the aorta to provide the desired bypass blood flow, and the downstream end (distal) of the dissected vessel will be connected to the target vessel in a distal anastomosis.

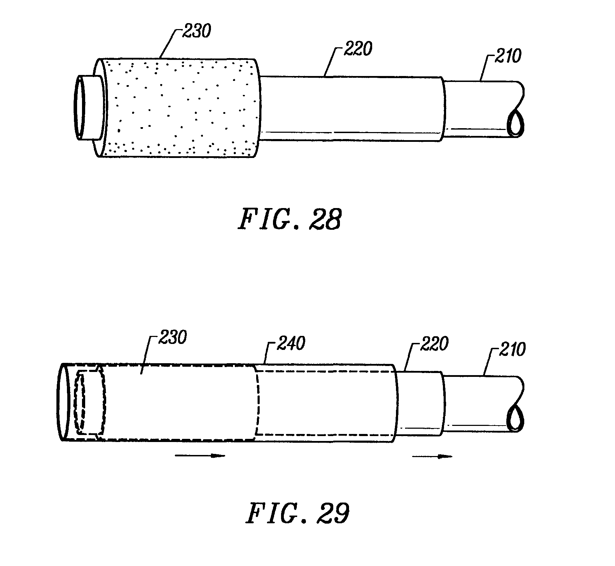

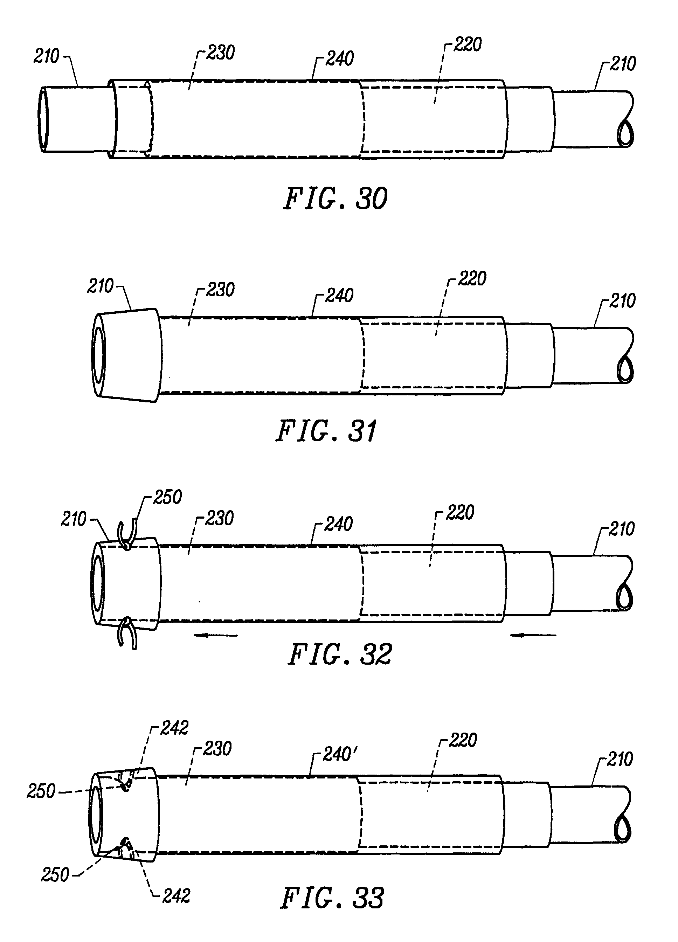

In order to perform an anastomosis with the fastener 208 of the present invention, the graft vessel 210 is first coupled to the fastener 208 by inserting a free end of the graft vessel 210 through an opening in the tubular member 220 with a conventional insertion device (not shown) and moving the graft vessel 210 longitudinally within the tubular member 220 until the free end of the graft vessel extends a short distance beyond an end of the tubular member as shown in Fig. 28. The fastener 208 with the graft coupling member 230 attached thereto is then inserted into an opening in a tubular introducer 240 which has an inner diameter smaller than an outside diameter of the graft coupling member 230 but larger than an outside diameter of the flexible tubular member 220 (Fig. 26). The introducer 240 preferably includes at least one longitudinal perforation (not shown) to allow for easy removal of the introducer as further described below. The inner wall of the introducer 240 will radially compress the graft coupling member 230 into its at least one compressed state. The free end of the graft vessel 210 is then everted over an end of the introducer 240 as shown in Fig. 31. The introducer 240 is then pulled back to a short distance over the fastener 208 (while holding tubular member 220) to a position in which at least an end portion of the graft coupling member 230 is exposed and extends a short distance from an end of the introducer 240 and engages with a portion of the graft vessel 210 in the everted configuration (Fig. 32). The natural adhesiveness of graft vessel 210 may be sufficient to secure the graft vessel 210 to the graft coupling member 230. If necessary, one or more sutures 250 may be applied to the graft vessel 210 and the graft coupling member 230 to secure the graft vessel 210 to the fastener 208 in the event that the natural adhesiveness and compressibility of the graft vessel 210 is insufficient to temporarily secure it to the graft coupling member 230. Alternatively, the graft vessel 210 can be secured to the graft coupling member 230 with biological glue, other adhesive means, by tying one or more sutures circumferentially around the graft vessel 210, or by any other suitable means.

An alternative embodiment of the tubular introducer 240 is shown in Figs. 27 and 33 and indicated at 240'. The introducer includes at least one groove 242 formed in one end of the wall of the introducer. One or more sutures 250 can be inserted through the groove 242 to secure the everted graft vessel 210 to the graft coupling member 230. It is to be understood that the introducer may have configurations other than those shown herein without departing from the scope of the invention.

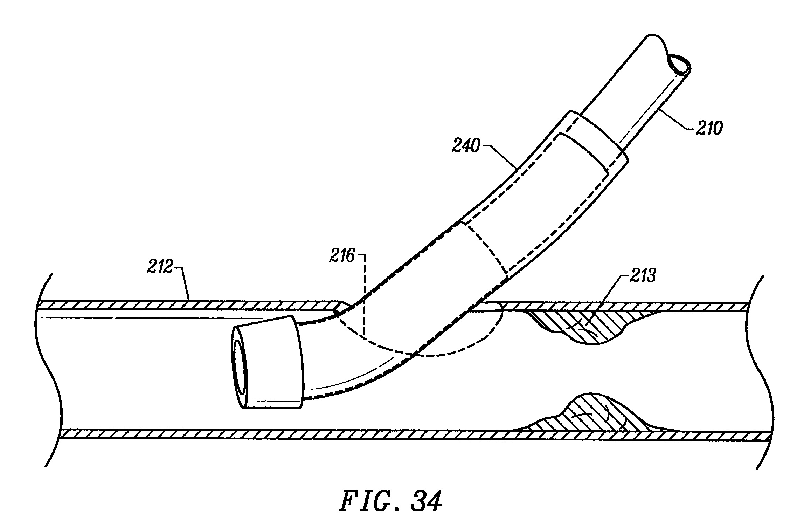

The introducer (240 or 240') is then introduced into the target vessel 212 through an incision (opening) 216 formed in a side wall of the target vessel 212 (Fig. 34). The incision 216 can be made with the use of a conventional scalpel or other appropriate cutting instrument. Alternatively, a circular or oval punch may be used to facilitate the arteriotomy. The fastener 208 is preferably positioned in the target vessel 212 via introducer 240, 240' such that at least an end portion of the graft coupling member 230 extends generally coaxial with the target vessel 212. With the fastener 208 securely positioned in the target vessel 212 via the introducer 240, 240', the introducer can be pulled back over the fastener to allow the graft coupling member 230 to radially expand back towards its normal expanded state to sealingly engage the fastener with an inner wall of the target vessel to complete the anastomosis. The introducer 240, 240' preferably will be a conventional peel-away introducer such as that manufactured by Modified Polymer Components, Inc. of Sunnyvale, California, so that it can be easily separated and removed from the fastener 208 and graft vessel 210.

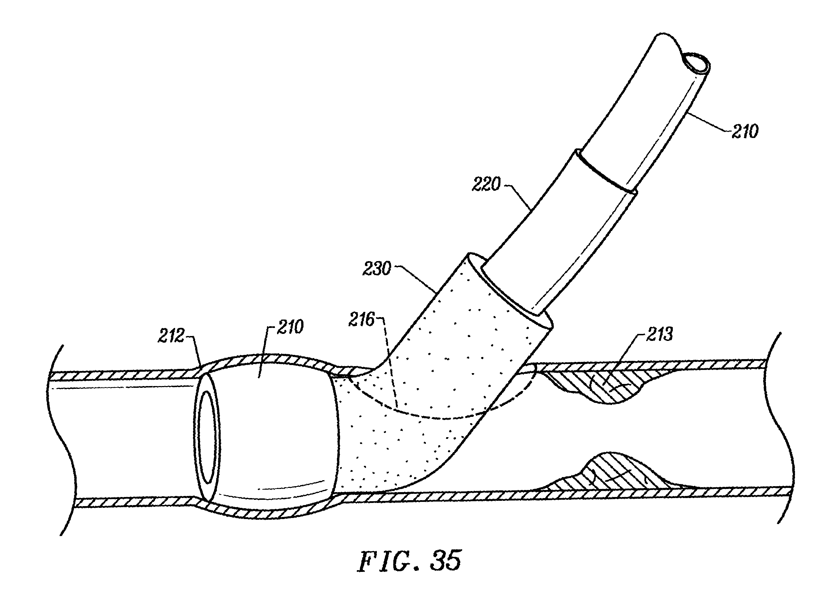

As shown in Fig. 35, the self-expanding nature of the graft coupling member 230 permits the graft coupling member 230 to radially expand to sealingly engage the graft vessel 210 with an inner wall of the target vessel 212 to complete the anastomosis. The engagement of the fastener 208 with the graft vessel 210 and inner wall of the target vessel 212 prevents substantial longitudinal movement of the fastener 208 within the target vessel. If necessary, one or more sutures can be applied to the anastomosis site to prevent the graft vessel 210 from being pulled out from the target vessel 212. The foam material forming the graft coupling member 230 will apply a gentle circumferentially uniform, radial pressure against the inverted graft vessel 210 and the inner wall of the target vessel 212. An intima-to-intima anastomosis results. The flexibility of the foam material and the tubular member 220 permits the fastener device 208 to be substantially compliant with the target vessel 212 and the graft vessel 210 to reduce the onset of thrombosis.

If required, cardiac stabilization such as described in co-pending patent application, serial number 09/131,075, for Compositions, Apparatus and Methods For Facilitating Surgical Procedures, filed August 7, 1998 and invented by Francis G. Duhaylongsod, M.D, may be used during the procedure. Other pharmacological or mechanical methods may also be used.

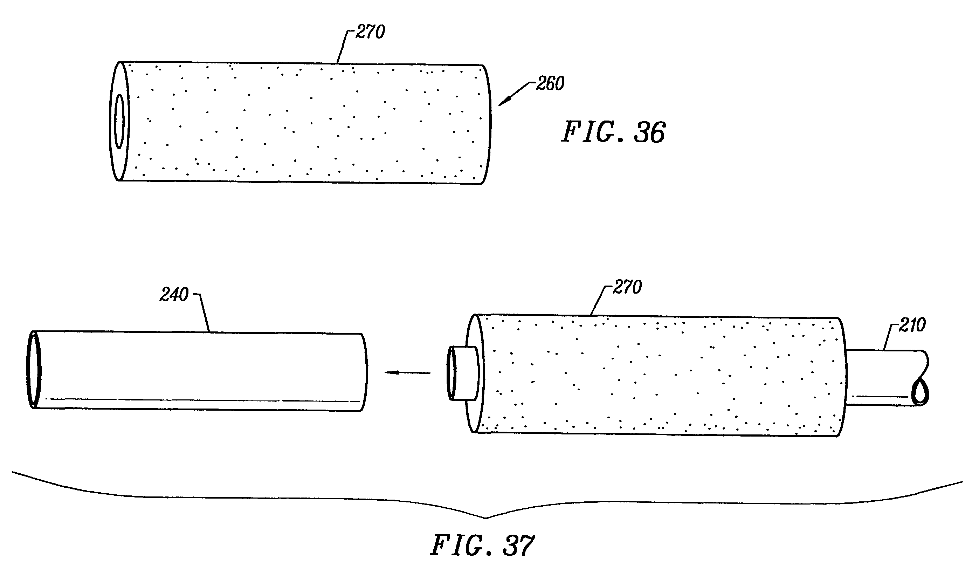

In an alternative embodiment of the fastener 208 (shown in Figs. 24-35), a fastener 260 (shown in Figs. 36-42) comprises a single, elongated tubular member 270 made from a radially compressible and radially self-expandable material, preferably a non-metallic foam material. The foam material is preferably sufficiently radially rigid to maintain its shape within the target vessel 212 to provide a fluid-tight seal, and should also be sufficiently flexible to be inserted through an incision 216 in the target vessel 212. The foam material in this embodiment is preferably foam such as manufactured by W.L. Gore of Arizona, for example. Alternatively, the foam may be a material having 100 pores per inch as described above. The outer diameter of the tubular member 270 in its free expanded state is preferably between about 10 to 80% larger than the inner diameter of the target vessel 212 into which it is inserted, and the inner diameter of the tubular member 270 in its free expanded state is preferably about 10% to 30% larger than the outer diameter of the graft vessel 210. The tubular member 270 is preferably between about 4.0 and 12.0 mm in length, and more preferably about 5.0 to 8.0 mm in length, for example.

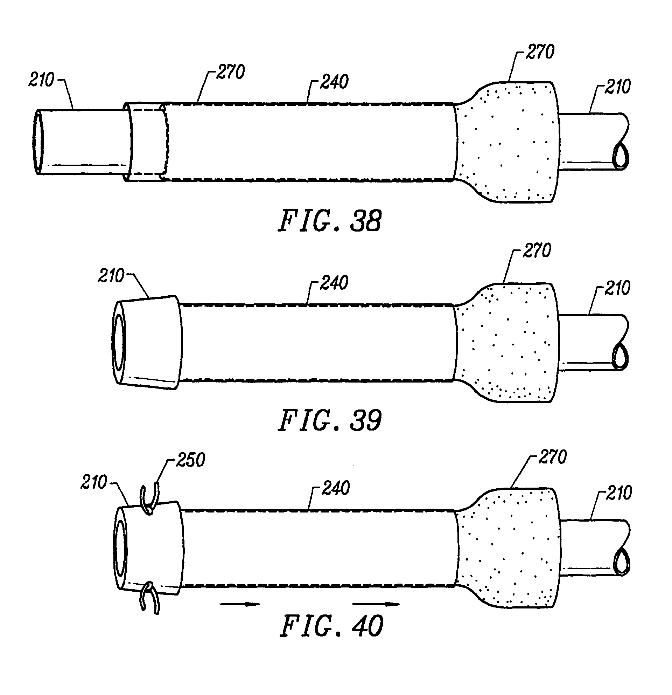

The graft vessel 210 may be coupled to the tubular member 270 and inserted into the target vessel 212 in substantially the same way as described above for the tubular member/graft coupling member combination of Figs. 24-35. Referring to Figs. 37-42, the foam tubular member 270 and graft vessel 210 is inserted into an opening in a tubular introducer 240 which has an inner diameter which is smaller than an outside diameter of the tubular member 270, as shown in Fig. 37. The inner wall of the introducer 240 will radially compress the tubular member 270 into a compressed state. A free end of the graft vessel 210 is then inserted through an opening in the tubular member 270 and everted over an end of the introducer 240 (Figs. 38 and 39). The introducer 240 can then be pulled back a short distance over the tubular member 270 (while holding tubular member 270) to a position in which at least an end portion of the tubular member 270 is exposed and extends a short distance from an end of the introducer 240 and engages a portion of the graft vessel 210 in the everted configuration (Fig. 40). One or more sutures 250 may be applied between the graft vessel 210 and the tubular member 270 to secure the graft vessel 210 to the tubular member 270. Alternatively, the graft vessel 210 may be secured to the tubular member 270 with biological glue, other adhesive means, by tying one or more sutures circumferentially around the graft vessel 210, or by any other suitable means.

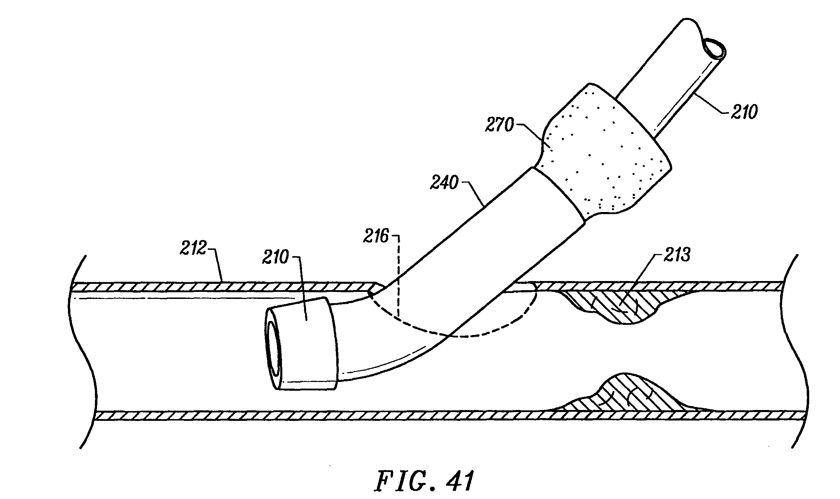

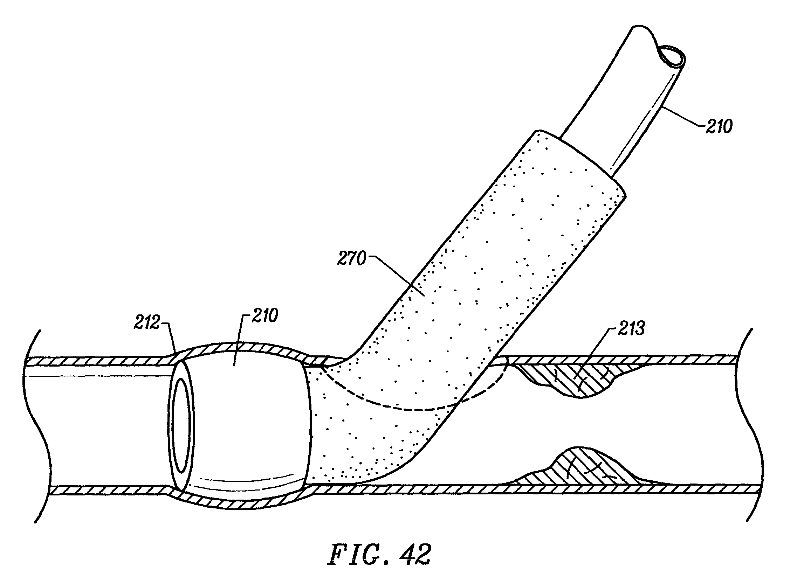

Alternatively, the tubular member 270 may be coupled to the graft vessel 210 by providing an introducer (not shown) as described above with reference to Fig. 33 having at least one groove in a wall of the introducer which extends axially from an end of the introducer. One or more sutures can be provided through the groove to secure the everted graft vessel 210 to the tubular member 270. The introducer 240 is then introduced into the target vessel 212 through an incision 216 in a wall of the target vessel 212 and pulled back over the tubular member 270 (Fig. 41). The tubular member 270 will then radially expand back towards its free, normally expanded state to sealingly join the graft vessel 210 to the target vessel 212 in a compliant, patent anastomosis (Fig. 42). If necessary, one or more stay sutures can be applied to the anastomosis site to prevent the graft vessel 210 from pulling out the target vessel 212. The introducer 240 can then be peeled away from the tubular member 270 and the graft vessel 210 to complete the anastomosis procedure.

It may be possible for a surgeon to radially compress the graft coupling member with his fingers or other appropriate surgical instrument without requiring the use of a separate tubular introducer. The surgeon can then deftly evert the graft vessel over the compressed graft coupling member (or one-piece tubular member) to couple them to one another. The surgeon can then use his fingers to continue to radially compress the graft coupling member as it is inserted into the target vessel through an incision formed therein. By removing his fingers from engagement with the graft coupling member, the graft coupling member will tend to radially expand back towards its normal expanded state to sealingly engage the graft vessel with an inner wall of the target vessel in a completed anastomosis. Similarly, other alternatives, substitutions, modifications and equivalents are possible without departing from the scope of the inventions described herein.

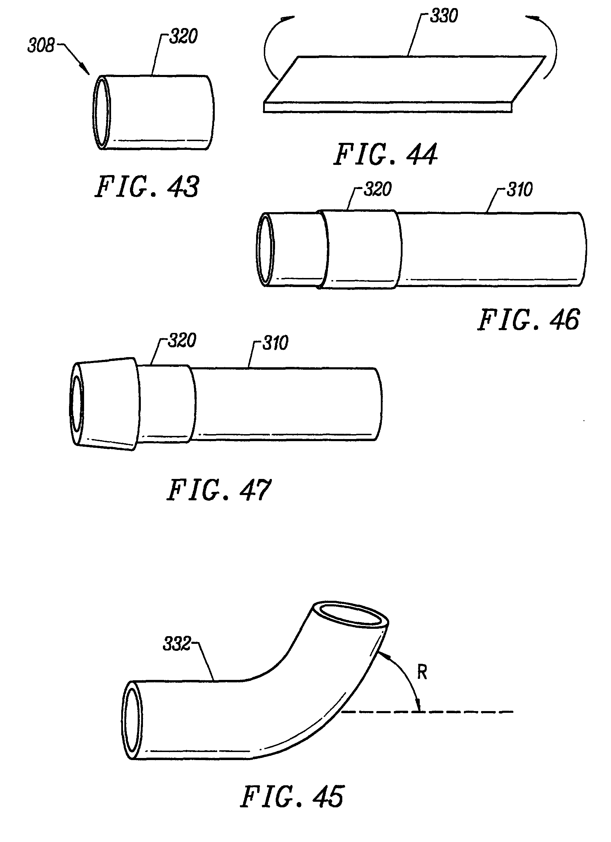

Referring now to Figs. 43-51, an alternate embodiment anastomosis device is shown and generally indicated with reference numeral 308. The anastomosis device (or fastener) 308 is used to connect a graft vessel 310, such as a thoracic artery or saphenous vein, to a target coronary vessel 312, such as the left descending artery, in an anastomosis. The anastomosis device 308 of the present invention may also be used in connecting various other vessels or arteries and may be used to connect synthetic vascular grafts to an artery.

The fastener 308 comprises a tubular member 320 as shown in Fig. 43. The tubular member 320 is constructed from a deformable material that must satisfy various criteria such as moldability, strength, biocompatability, and light absorption characteristics. The deformable material may comprise a material that becomes moldable in vivo by a heat-activated process upon the application, for example, of radiant energy from an energy source such as a radio frequency energy source, microwave energy source, ultrasonic energy source, or light energy source at a predetermined frequency, wavelength or wavelengths. Alternatively, the deformable material may become moldable by other conventional heating means, such as by conductive heating or by convective heating. In addition, deformable materials that become moldable by a non-thermal light-activated process without generating heat, such as by a photochemical process or a photophysical process (i.e., photoacoustic or plasma formation), also are contemplated for use in the present invention.

The deformable material should become moldable or fluent at a condition such as temperature that is not significantly injurious to tissue or surrounding fluids if maintained at that condition for the amount of time needed to implant and shape the material. Additionally, if temperature is the germane condition, the material should become moldable at a temperature above about 40 degrees C, since that temperature is greater than a person's body temperature with hyperthermia or fever (approximately 38 to 40 degrees C). The minimum molding temperature prevents the material from spontaneously softening or melting in response to elevated, physiologically occurring body temperatures.

It is also preferred that the deformable material have a substantially crystalline or semi-crystalline structure so that when irradiated and transformed into its moldable, fluent state, it will undergo a rapid transition to a viscous fluid that will flow readily, yet remain cohesive, when subjected to molding forces. The materials used in this invention are termed "fluent" when in their moldable state. The actual viscosity of the fluent material that allows the material to be molded without significant mechanical disruption of the tissue depends on the particular tissue and the method by which the material is molded. In general, it is preferred that the material be such that once rendered fluent, the material may be shaped or formed using a physiologically acceptable amount of force to reduce damage to surrounding tissue during the molding process. The material must also be structurally sound in its non-fluent, or solid form, to provide mechanical support and strength to withstand forces exerted upon the shaped material during its functional lifetime in vivo at the anastomosis site. This requirement is important if the material is also bioerodable after its functional lifespan. The material can include one or more predefined perforations or apertures (not shown) once transformed from a delivery configuration to its final, shaped support configuration. The perforations may allow increased flexibility to facilitate delivery and reduce tissue erosion during and after implementation, and increase ingrowth of tissue for anchoring and encapsulation of the material.

Where light energy is used as the heat activating medium (i.e., for a photothermal process), the deformable material should preferably absorb light within a wavelength range that is not absorbed significantly (from a clinical perspective) by tissue, blood, physiological fluids, or water. Wavelengths in the ultraviolet, visible, and infrared spectrum may be used, for example, to selectively heat the material to its molding temperature. Ultraviolet light typically has a wavelength of between about 100 and 400 nm, visible light has a wavelength typically in the range of between about 400 and 700 nm, and infrared light typically has a wavelength of between about 700 and 15,000 nm. Additionally, a chromophore such as a dye or pigment may be incorporated into the material to selectively absorb light at a predefined, specific wavelength. As an alternative to compounding the material with a chromophore, polymers or copolymers that naturally absorb the wavelength spectrum of the light may be used and the mechanism of action can be either photothermal or photochemical as explained above. Preferably, one or more of a wide variety of therapeutically useful pharmacological agents may be impregnated into the material, thus providing local drug delivery to prevent thrombus formation, smooth muscle cell proliferation, or inflammatory responses. Examples of such drugs include anti-platelet or anti-thrombus agents (such as Heparin, Hirudin, tPA, Streptokinase, Urokinase, Persantine, Aspirin, etc.), anti-inflammatory agents (such as steroidal and non-steroidal compounds), and anti-proliferative compounds (such as suramin, monoclonal antibodies for growth factors, and equivalents). In addition, other potentially useful drugs can be impregnated into the material to facilitate healing and reduce the incidence of thrombosis at the anastomosis site, such as immunosuppressant agents, glycosaminoglycans, collagen inhibitors, and endothelial cell growth promoters.

The deformable material is also preferably bioerodable. By "bioerodable", it is meant that the material will be broken down in the body and gradually absorbed or eliminated by the body after its functional lifespan, which in the case of the structural support application of the present invention preferably is between 3 to 24 months, although shorter or longer periods may be appropriate depending on the particular application for the fastener 8. Once the material has been absorbed by the body, the graft will exhibit similar compliance to that of the native artery. The new tissue ingrowth forms a natural biological field between the graft vessel and the target vessel. The new tissue growth connects the graft vessel to the target vessel so that the fastener is no longer required.

Examples of deformable materials which may be used in the present invention and which typically satisfy the above criteria include suitable polymers and copolymers, or combinations thereof, such as polyglycotic/polylactic acid (PGLA), polyhydroxybutylate valerate (PHBV), polycaprolactone (PCL), polycaprolactone homopolymers and copolymers, and the like. Many of these materials (and other similar materials) are fully described in United States Patent No. 5,662,712 to Pathak et al..

Polycaprolactone homopolymers and copolymers, for example, possess adequate strength in their solid form to structurally support soft tissue lumens. Additionally, once positioned and molded to a desired shape in a body lumen or about a vessel, the physical structure of such materials is sufficiently nonvariable, in the period prior to their bioerosion, to maintain constant dimensions in their molded state. Polycaprolactones have a crystalline melting point of approximately 60 degrees C, and can be deployed in vivo using the method described in detail below. Additionally, such polymeric materials in their fluent state are well adapted for mechanical deformation to various degrees and into various configurations. Polcaprolactone homopolymers and copolymers can be designed to resorb as soon as three months after implantation, which may be preferable for the application of the fastener 308. For example, polycaprolactone copolymerized with lactic or glycolic acids may resorb over a 3 to 9 month period. Additionally, other bioabsorbable, deformable materials which have higher melting temperatures, such as polyglycolides and polylactides, may be used since these materials have glass transition temperatures on the order of about 45 degrees C which makes them moldable at physiologically acceptable temperatures. These examples are in no way meant to be limiting, however, and any deformable, moldable material that satisfies the criteria described above may be used in the present invention without departing from the scope of the invention. Any of the methods known in the art of polymer processing may be used to form the polymeric material into the tubular shape of Fig. 43 and, if necessary, to compound chromophores into the material.

The diameter of the tubular member 320 will vary depending on the size of the graft vessel about which it is positioned. Preferably. the inner diameter of the tubular member 320 will generally be between about 0.5 to 6.0 mm for a coronary anastomosis. The length of the tubular member 320 can also vary. and is preferably between 4 and 20 mm in length, for example. Alternatively, as shown in Fig. 44, the fastener 308 may comprise a relatively thin sheet of material 330 that can be conformed about an external surface of the graft vessel 310 prior to the anastomosis procedure described below. The sheet 330 may be rolled about the graft vessel 310. The adhesiveness of the material allows the edges of the sheet 330 to adhere to one another. If required. additional adhesive may be applied to one or both of the edges of the sheet. Upon irradiation and subsequent expansion of the material, the sheet 330 will be caused to unroll to press the graft vessel 310 into conforming contact with the target vessel 312. Further alternatively, as shown in Fig. 45, the fastener 308 may comprise a pre-shaped tubular member 332 which will at least have a first bend along its length such that a portion of the tubular member extends at an angle "R" of between about 30° and 90° from a longitudinal centerline. Pre-shaped tubular member 332 provides support for the graft vessel through the anastomosis site after employment of the device to prevent kinking of the graft vessel.

Figures 46-51 show an exemplary use of the anastomosis device 308 of the present invention in an open surgical coronary artery bypass graft procedure via a median or partial sternotomy. The anastomosis device 308 of this example is preferably formed from a heat-activated deformable material, although a non-thermal light-activated deformable material can be used as well without departing from the scope of the invention. This example is meant to be by illustration only, and in no way is meant to be limiting. The present invention can be used in other cardiac surgery procedures such as minimally invasive direct coronary artery bypass grafting (MIDCAB) on a beating heart though a small incision (thoracotomy) (about 6-8 cm) in the left side of the chest wall, in endoscopic minimally invasive cardiac surgery bypass graft procedures, and in other vascular procedures to join two vessels together. By way of example, the left internal thoracic artery is used as the graft vessel 310. In this example, the left anterior descending artery is used as the target vessel 312 and contains a build-up of plaque or narrowing 313. If left untreated, this diseased artery may lead to insufficient blood flow and eventual angina, ischemia, and possibly myocardial infarction.

Conventional coronary bypass graft procedures require that a source of arterial blood be prepared for subsequent bypass connection to the diseased artery. An arterial graft can be used to provide a source of blood flow, or a free vessel graft may be used and connected at the proximal end to a source of blood flow. Preferably, the source of blood flow is any one of a number of existing arteries that are dissected in preparation for the bypass graft procedure. In many instances, it is preferred to use either the left or right internal thoracic artery. In multiple bypass procedures, it may be necessary to use free graft vessels such as the saphenous vein, gastroepiploic artery in the abdomen, and other arteries harvested from the patient's body as well as synthetic graft materials, such as Dacron or Gortex grafts. If a free graft vessel is used, the upstream end (proximal) of the dissected vessel, which is the arterial blood source, will be secured to the aorta to provide the desired bypass blood flow, and the downstream end (distal) of the dissected vessel will be connected to the target vessel in a distal anastomosis.

In order to perform an anastomosis with the fastener 308 of the present invention, the graft vessel 310 preferably is first coupled to the fastener 308. Preferably, the graft vessel 310 is coupled to the fastener 308 by first inserting a free end of the graft vessel 310 through an opening in the tubular member 320 and moving the graft vessel 310 longitudinally within the tubular member 320 until the free end of the graft vessel extends a short distance beyond an end of the tubular member as shown in Fig. 46. Preferably, the free end of the graft vessel 310 is then everted over an end of the tubular member 320 as shown in Fig. 47. The natural adhesiveness of graft vessel 310 or tubular member 320 may be sufficient to secure the graft vessel 310 to the tubular member 320. If necessary, one or more sutures can be applied between the graft vessel 310 and the tubular member 320 to secure the graft vessel 310 to the fastener 308 in an everted configuration. Alternatively, the graft vessel 310 can be secured to the tubular member 320 with glue, other adhesive means, by tying one or more sutures circumferentially around the graft vessel 310, or by any other suitable means.

Where light energy is used as the heat activating medium, a suitable light-diffusing balloon catheter device 350 which has the ability to deliver light energy to luminal surfaces such as blood vessels is inserted through the lumen of the graft vessel 310 and fastener 308. An example of a suitable light-diffusing balloon catheter device 350 is shown in United States Patent No. 5,441,497 to Narciso et al., although other suitable light-diffusing balloon catheter devices may also be used, such as that disclosed in Spears United States Patent No. 4,773,899, for example. Additionally, a separate light diffusing catheter (or guidewire) and balloon catheter (not shown) may be used in conjunction with one another, as disclosed, for example, in Spears U.S. Patent No. 5,199,951 . Generally, the light-diffusing balloon catheter 350 includes a light diffusing guidewire 360 which is used in conjunction with an inflated balloon 362. The balloon 362 is affixed to the guidewire 360 so that the balloon 362 overlies the light diffusing member 364 of the guidewire 360. The wall of the balloon 362 is transparent at the wavelength of light being delivered to (or received from) the surrounding tissue. At least one optical fiber 366 delivers light from an external light source (not shown) to the light diffusing member 364. The light diffusing member 364 within balloon 362 is selected for optimum transmission of light with maximum light scattering.

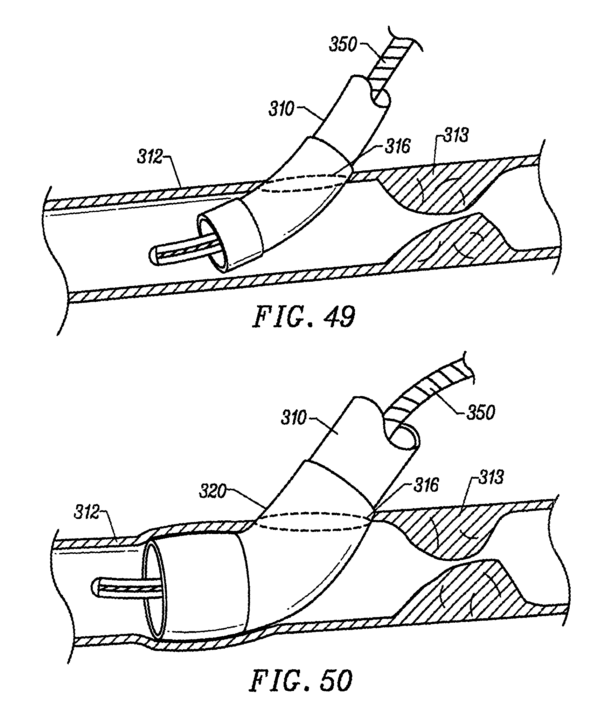

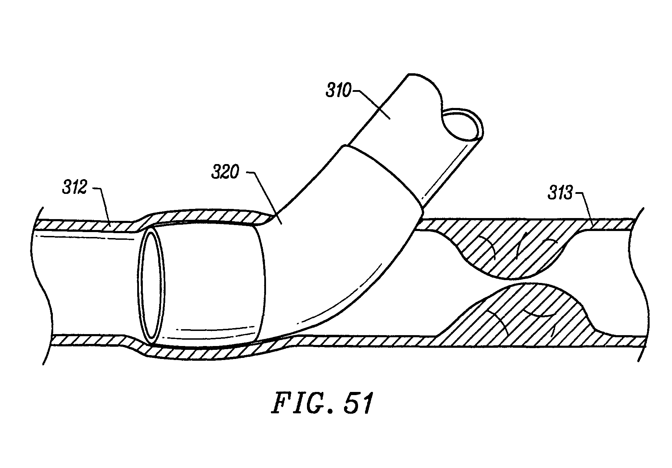

The graft vessel 310 is inserted into the target vessel 312 through an incision (opening) 316 in a wall of the target vessel 312. The fastener 308 is preferably positioned in the target vessel 312 such that at least an end portion of the tubular member 320 extends generally coaxial with the target vessel 312 (Fig. 49). With the fastener 308 securely positioned in the target vessel 312, light energy at a given wavelength or wavelengths is supplied to the light diffusing member 364 from the energy source via optical fiber 366 to irradiate, or illuminate, the tubular member 320 with light at a wavelength or wavelengths at which the deformable material readily absorbs. Upon absorption of the light energy, the deformable material forming tubular member 320 is transformed into its moldable state. Alternatively, where heat energy is used as the heat activating medium, the deformable material can be made fluent by use of a suitable thermal balloon catheter (not shown) in lieu of the light-diffusing balloon catheter 350, or by any other conductive or convective heating means as would be obvious to one of ordinary skill in the art, such as by providing a heated saline irrigation flush. Inflation of the balloon 362 causes the tubular member 320 to radially expand outwardly, thereby pressing the graft vessel 310 into conforming engagement with an inner wall of target vessel 312 (Fig. 50). Alternatively, where the deformable material comprises a rolled sheet 330 such as in Fig. 44 which can be reconfigured prior to molding, the material is reconfigured using the balloon and then irradiated to transform it into its moldable state to mold it into conformance with the everted graft vessel 310 and target vessel 312. By discontinuing the supply of light energy from the energy source, the deformable material will become non-fluent and remain in its molded configuration. The balloon 362 is then deflated and the catheter device 350 withdrawn from the graft vessel 310 (Fig. 51).

The engagement of the graft vessel 310 via tubular member 320 with the inner wall of the target vessel 312 prevents substantial longitudinal movement of the tubular member 320 within the target vessel. The tubular member 320 in its molded configuration will apply a gentle uniform, circumferential pressure against the everted graft vessel 310 and the inner wall of the target vessel 312. An intima-to-intima anastomosis results. The flexibility of the tubular member 320 permits the fastener device 308 to be substantially compliant with the target vessel 312 and the graft vessel 310 to reduce thrombosis formation. Additionally, the tubular member 320 is preferably bioerodable, so that after its functional lifespan (e.g., 3 to 24 months), it will degrade and leave remaining a natural patent, sealed, compliant anastomosis.

If required, cardiac stabilization such as described in co-pending patent application for Compositions, Apparatus and Methods For Facilitating Surgical Procedures, filed August 7, 1998 and invented by Francis G. Duhaylongsod, M.D, (referred to above) may be used during the procedure. Other pharmacological or mechanical methods may also be used.

In an alternative embodiment of the present invention, a different fastener device is disclosed for sealingly joining a graft vessel to a target vessel at an anastomosis site. The fastener (not shown) of this embodiment comprises a coating of a fluent, curable material, such as a liquid or viscous gel, which is applied to an external surface of a free end portion of the graft vessel 310. Examples of suitable curable materials include, but are not limited to, light-curable materials such as the chemical class of biocompatible compounds including acrylate polymers which can be cured when exposed to ultraviolet light, and acrylate urethane polymers which can be cured when exposed to ultraviolet light and/or visible light of sufficient intensity. These materials also can be combined with a dye that absorbs light at a very specific wavelength so that light energy can be used to selectively and rapidly cure the material and not heat the surrounding tissue.

Other suitable light-curable materials may include bioerodable hydrogels which can be photopolymerized (or gelled) in vivo by a brief exposure to long wavelength ultraviolet light, such as polyethylene-glycol (PEG) based hydrogels as fully disclosed in United States Patent No. 5,410,016 to Hubbell et al. Several biocompatible, photopolymerizable macromer hydrogels are disclosed in United States Patent. No. 5,410,016 (see, for example, Table I therein) which are suitable as tissue supports by forming shaped articles within the body upon the application of light energy at a specific wavelength. These macromers, for example, can be composed of degradable comonomers such as glycolides, lactides, and caprolactones of various molecular weights and compositions. These materials are given by way of example only, and in no way are meant to limit the invention to the specific materials disclosed. Any suitable light-curable material having the requisite strength, biocompatability and moldability criteria may be used without departing from the scope of the present invention. In addition, heat- curable materials can be used in a similar fashion with the method of heating chosen from the list set forth above for deformable materials, such as convective or conductive heating.

As in the previous example, the curable material can be impregnated with one or more anti-platelet or anti-thrombus agents, anti-inflammatory agents, and anti-proliferative compounds. In addition, other potentially useful drugs can be impregnated into the material to facilitate healing and reduce the incidence of thrombosis at the anastomosis site, such as immunosuppressant agents, glycosaminoglycans, collagen inhibitors, and endothelial cell growth promoters. Preferably, where light-curable materials are used, wavelengths in the ultraviolet, visible, and infrared light spectrum may be used, for example, to transform the curable material into its cured state, since light energy within these wavelengths is not significantly injurious to surrounding tissues. Additionally, a chromophore such as a dye or pigment may be incorporated into the material to selectively absorb light at a predefined, specific wavelength or wavelengths.

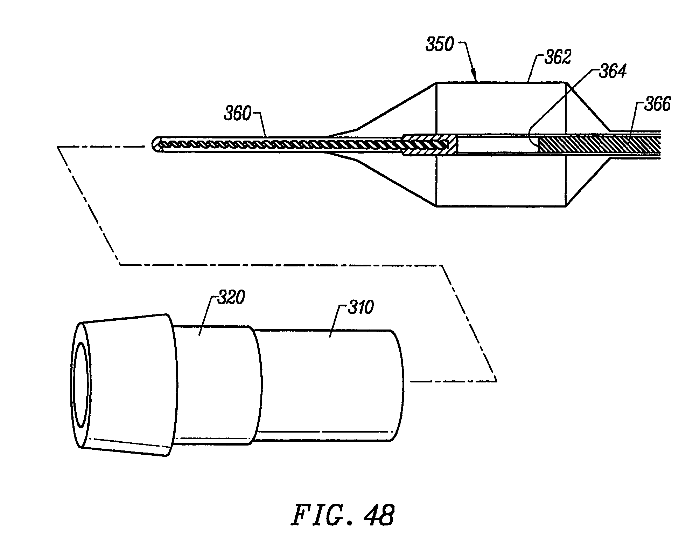

The method of using the fastener of this embodiment is similar in many respects to that shown for use of the tubular member 320 of Figs. 43-51, with the principal difference being that the energy supply and balloon expanding steps are typically reversed. In this alternative embodiment, after the coating of curable material is applied to an external surface of the free end portion of the graft vessel 310, the free end portion of the graft vessel 310 is everted. The curable material typically has a natural adherent property in which case the free end portion of the graft vessel 310 in its everted configuration will be adhered and secured to the coating material. If necessary, one or more sutures may be required to retain the free end of the graft vessel 310 in an everted configuration. Subsequently, where a light-curable coating material is used, a light-diffusing balloon catheter 350 such as shown in Fig. 48 preferably is inserted into the graft vessel 310. Alternatively, as above, a separate light diffusing catheter (or guidewire) and balloon catheter (not shown) may be used in conjunction with one another.

At least a portion of the everted free end portion of the graft vessel 310 is then positioned in the target vessel 312 through an incision 316 in the target vessel 312. The balloon 362 of light-diffusing balloon catheter 350 is then inflated to radially expand at least the free end portion of the graft vessel 310 into conforming engagement with an inner wall of the target vessel 312. Once expanded, curing is achieved by irradiating, or illuminating, the free end portion of the graft vessel 310 with light energy at a predetermined wavelength or wavelengths supplied by an energy source coupled to the light diffusing member 364 of light-diffusing balloon catheter 350. The light energy preferably has a wavelength and intensity which does not have a significant adverse effect on the surrounding tissue, such as light within the ultraviolet, infrared, or visible light spectrum. The intensity of the light energy is sufficient to transform the curable material into its cured, non-fluent state to complete the anastomosis. The balloon 362 can then be deflated and the light-diffusing balloon catheter 350 removed from the graft vessel 310. Again, an intima-to-intima anastomosis results which reduces the possibility of thrombosis formation at the anastomosis site. Alternatively, where a heat-curable coating material is used, the curable material can be cured by use of a suitable thermal balloon catheter (not shown) in lieu of the light-diffusing balloon catheter 350, or by any other conductive or convective heating means as would be obvious to one of ordinary skill in the art, such as by providing a heated saline irrigation flush.

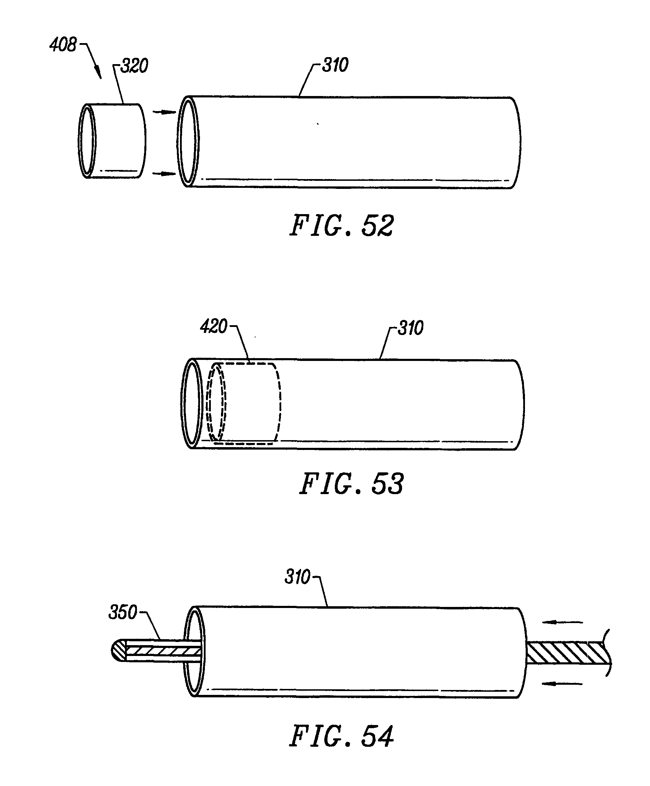

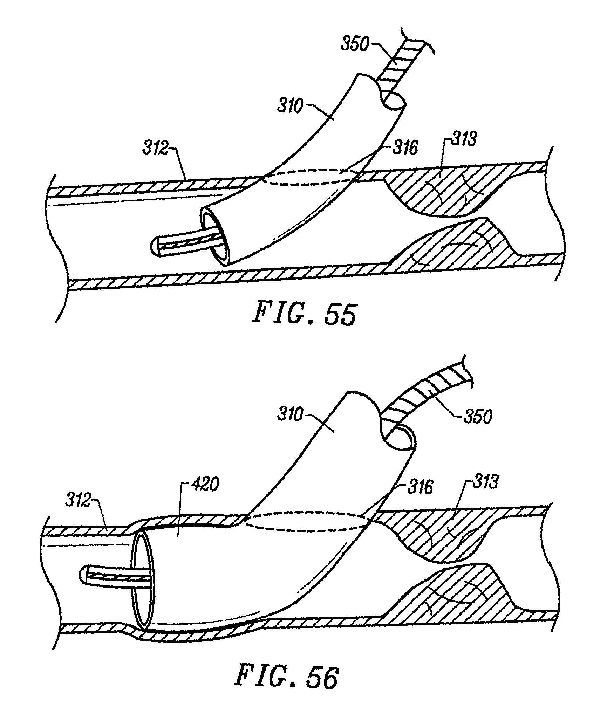

In alternative embodiments of the invention, the anastomosis fastener can comprise either a tubular member formed of a deformable material or a coating of a curable material that is applied to the internal wall of a free end portion of graft vessel 310. In the case of the embodiment shown in Figs. 52-57, the fastener 408 comprises a tubular member 420 having a diameter sized to permit the tubular member 420 to be inserted longitudinally into the graft vessel 310, as shown in Figs. 52-53. As shown in Fig. 54, where a light-activated deformable material is used, the light-diffusing balloon catheter 350 can then be inserted into the graft vessel 310 and the tubular member 420 and positioned such that the balloon (not shown) of the light-diffusing balloon catheter 350 is adjacent an internal surface of tubular member 420. If necessary, the balloon can be partially inflated to secure the tubular member 420 in place prior to inserting the graft vessel 310 into the target vessel 312. With the tubular member 420 securely in place within the free end portion of the graft vessel 310, the graft vessel 310 is then inserted into the target vessel 312 such that at least the free end portion of the graft vessel 310 extends generally coaxial with the target vessel 312, as shown in Figs. 55-56.

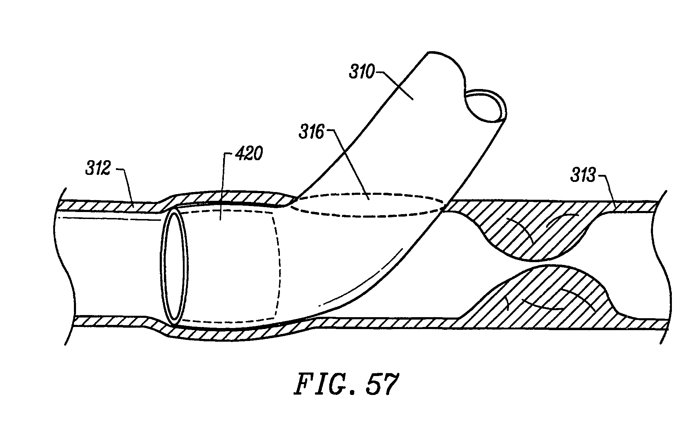

With the graft vessel 310 securely positioned in the target vessel 312, light at a given wavelength or wavelengths is supplied by the light-diffusing balloon catheter 350 to irradiate, or illuminate, the tubular member 420 with light at a wavelength or wavelengths at which the material readily absorbs. Upon absorption of the light, the material forming tubular member 420 is irradiated to transform it into its fluent, moldable state. Further inflation of the balloon causes the moldable tubular member 420 to radially expand outwardly, thereby pressing the graft vessel 310 into conforming engagement with an inner wall of target vessel 312. By discontinuing the supply of light energy from the light source, the formable material will become non-fluent and remain in its molded configuration. The balloon is then deflated and the catheter device 350 withdrawn from the graft vessel 310, as shown in Fig. 57. An intima-to-adventitia anastomosis results.

Alternatively, a coating of a curable material can be applied to an internal wall of the free end portion of the graft vessel 310. In this particular embodiment, the balloon 362 will be expanded fully prior to applying light energy, or heat, to the coating material. Where a light-curable coating material is used, with the graft vessel in conforming engagement with the target vessel 312, curing is achieved by irradiating, or illuminating, the free end portion of the graft vessel 310 with light energy at a predetermined wavelength or wavelengths supplied by an energy source coupled to the light diffusing member of the light-diffusing balloon catheter 350. The intensity of the light energy is sufficient to transform the curable material into its cured, non-fluent state to complete the anastomosis. The balloon can then be deflated and the light-diffusing balloon catheter 350 removed from the graft vessel 310.

The engagement of the graft vessel 310 via tubular member 420 (or the cured, non-fluent coating of curable material) with the inner wall of the target vessel 312 prevents substantial longitudinal movement of the graft vessel 310 within the target vessel 312. The tubular member 420 (or the cured, non-fluent coating) in its molded configuration will apply a gentle uniform, circumferential pressure against the graft vessel 310 and the inner wall of the target vessel 312. The flexibility of the tubular member 420 (or the cured, non-fluent coating) permits the fastener device 408 to be substantially compliant with the target vessel 312 and the graft vessel 310 to reduce thrombosis formation. Additionally, the tubular member 420 (or the cured, non-fluent coating) is preferably bioerodable, so that after its functional lifespan (e.g., 3 to 24 months), it will degrade and leave remaining a natural patent, sealed anastomosis. Although the embodiments of Figs. 52-57 result in an intima-to-adventitia anastomosis as opposed to an intima-to-intima anastomosis as in the above embodiment of Figs 43-51, the anastomosis of these embodiments results in a larger target vessel inner diameter over the previous embodiments, thus increasing the blood flow area, rather than reducing the diameter of the blood flow passage.

It will be observed from the foregoing that the anastomosis devices of the present invention have numerous advantages. Importantly, the devices require a minimal amount of manipulation and can be quickly installed. The devices have very few parts and are non-complex, thus simplifying attachment of the device to the vessels. The risk of thrombosis is reduced by substantially eliminating or reducing the exposure of the blood flow to foreign material. Furthermore, the devices enlarge the diameter of the artery, thus increasing the cross-sectional area of the blood flow passage, rather than reducing the diameter of the passage, as is common with prior art devices.

## Claims

1. An anastomosis device (10) for use in coupling an end of a first vessel (12) to a side of a second vessel (14), the second vessel (14) having an opening (18) formed in a side wall thereof for insertion of the device, the device (10) comprising a tubular member (43) at least a portion thereof being radially expandable, **characterised by** the tubular member (43) being preformed with a bend along its central longitudinal axis so that a portion (49) of the tubular member (43) extends out from the opening (18) in the side wall of the second vessel (14) while an end portion of the tubular member (47) extends generally coaxially with the second vessel (14) when the tubular member (43) is inserted into the second vessel (14), the tubular member (43) being sufficiently rigid in its preformed configuration to substantially retain its bent shape after the tubular member (43) is expanded.

2. The anastomosis device of claim 1 wherein the outer diameter of the tubular member (43) in a compressed state is less than 2 mm.

3. The anastomosis device of claim 1 or 2 wherein the outer diameter of the portion (49) of the tubular member (43) in an expanded state is greater than 2 mm.

4. The anastomosis device of claim 1, 2 or 3 wherein the length of the tubular member (43) is between 6 and 20 mm.

5. The anastomosis device of any of claims 1 to 4 wherein the tubular member (43) is formed from a shape memory alloy.

6. The anastomosis device of claim 1 wherein the tubular member (43) comprises a metal mesh material.

7. The anastomosis device of any one of the preceding claims wherein a side wall of the tubular member (43) extends substantially around the circumference of the tubular member (43) along the entire length thereof.

8. The anastomosis device of claim 7 wherein the tubular member (43) is bent at an angle of between 30 and 60 degrees relative to the central longitudinal axis of the tubular member (43).

9. The anastomosis device of claim 4 wherein the tubular member (43) is formed from nitinol.

## Patentansprüche

1. Anastomose-Einrichtung (10) zur Verwendung beim Verbinden eines Endes eines ersten Gefäßes (12) mit einer Seite eines zweiten Gefäßes (14), wobei das zweite Gefäß (14) eine Öffnung (18), die in einer Seitenwand davon gebildet ist, zum Einführen der Einrichtung hat, wobei die Einrichtung (10) ein röhrenförmiges Element (43) aufweist, von dem zumindest ein Abschnitt radial expandierbar ist,
**dadurch gekennzeichnet, dass** das röhrenförmige Element (43) mit einer Biegung entlang seiner zentralen Längsachse vorgeformt ist, sodass ein Abschnitt (49) des röhrenförmigen Elements (43) aus der Öffnung (18) in der Seitenwand des zweiten Gefäßes (14) heraus erstreckt, während ein Endabschnitt des röhrenförmigen Elements (47) sich im Wesentlichen koaxial zu dem zweiten Gefäß (14) erstreckt, wenn das röhrenförmige Element (43) in das zweite Gefäß (14) eingeführt wird, wobei das röhrenförmige Element (43) ausreichend rigide in seiner vorgeformten Konfiguration ist, um seine gebogene Form im Wesentlichen beizubehalten, nachdem das röhrenförmige Element (43) expandiert ist.

2. Anastomose-Einrichtung nach Anspruch 1, wobei der äußere Durchmesser des röhrenförmigen Elements (43) in einem komprimierten Zustand weniger als 2 mm beträgt.

3. Anastomose-Einrichtung nach Anspruch 1 oder 2, wobei der äußere Durchmesser des Abschnitts (49) des röhrenförmigen Elements (43) in einem expandierten Zustand größer als 2 mm ist.

4. Anastomose-Einrichtung nach Anspruch 1, 2 oder 3, wobei die Länge des röhrenförmigen Elements (43) zwischen 6 und 20 mm beträgt.

5. Anastomose-Einrichtung nach einem der Ansprüche 1 bis 4, wobei das röhrenförmige Element (43) aus einer Formgedächtnis-Legierung gebildet ist.

6. Anastomose-Einrichtung nach Anspruch 1, wobei das röhrenförmige Element (43) ein Metall-Netzmaterial aufweist.

7. Anastomose-Einrichtung nach einem der vorhergehenden Ansprüche, wobei eine Seitenwand des röhrenförmigen Elements (43) sich im Wesentlichen um den Umfang des röhrenförmigen Elements (43) erstreckt, entlang der gesamte Länge davon.

8. Anastomose-Einrichtung nach Anspruch 7, wobei das röhrenförmige Element (43) um einen Winkel zwischen 30 und 60 Grad relativ zu der zentralen Längsachse des röhrenförmigen Elements (43) gebogen ist.

9. Anastomose-Einrichtung nach Anspruch 4, wobei das röhrenförmige Element (43) aus Nitinol geformt ist.

## Revendications

1. Dispositif d'anastomose (10) destiné à être utilisé pour raccorder une extrémité d'un premier vaisseau (12) à un côté d'un deuxième vaisseau (14), le deuxième vaisseau (14) comportant une ouverture (18) formée dans une paroi latérale de celui-ci pour l'insertion du dispositif, le dispositif (10) comprenant un élément tubulaire (43) dont au moins une partie peut se dilater radialement, **caractérisé en ce que** l'élément tubulaire (43) est préformé avec une courbure le long de son axe longitudinal central de telle sorte qu'une partie (49) de l'élément tubulaire (43) s'étende hors de l'ouverture (18) dans la paroi latérale du deuxième vaisseau (14), tandis qu'une partie d'extrémité de l'élément tubulaire (47) s'étend de façon globalement coaxiale vis-à-vis du deuxième vaisseau (14), lorsque l'élément tubulaire (43) est inséré dans le deuxième vaisseau (14), l'élément tubulaire (43) étant suffisamment rigide dans sa configuration préformée pour conserver sensiblement sa forme incurvée après que l'élément tubulaire (43) ait été dilaté.

2. Dispositif d'anastomose selon la revendication 1, dans lequel le diamètre extérieur de l'élément tubulaire (43) dans un état comprimé est inférieur à 2 mm.

3. Dispositif d'anastomose selon la revendication 1 ou 2, dans lequel le diamètre extérieur de la partie (49) de l'élément tubulaire (43) dans un état dilaté est supérieur à 2 mm.

4. Dispositif d'anastomose selon la revendication 1, 2 ou 3, dans lequel la longueur de l'élément tubulaire (43) est comprise entre 6 et 20 mm.

5. Dispositif d'anastomose selon l'une quelconque des revendications 1 à 4, dans lequel l'élément tubulaire (43) est formé à partir d'un alliage à mémoire de forme.

6. Dispositif d'anastomose selon la revendication 1, dans lequel l'élément tubulaire (43) comprend un matériau en treillis métallique.

7. Dispositif d'anastomose selon l'une quelconque des revendications précédentes, dans lequel une paroi latérale de l'élément tubulaire (43) s'étend sensiblement autour de la circonférence de l'élément tubulaire (43) le long de la totalité de la longueur de celui-ci.

8. Dispositif d'anastomose selon la revendication 7, dans lequel l'élément tubulaire (43) est incurvé selon un angle compris entre 30 et 60 degrés par rapport à l'axe longitudinal central de l'élément tubulaire (43).

9. Dispositif d'anastomose selon la revendication 4, dans lequel l'élément tubulaire (43) est formé en Nitinol.
